(19) Europäisches Patentamt
European Patent Office
Office européen des brevets

(11) Veröffentlichungsnummer: **0 056 575**
**A1**

# (12) EUROPÄISCHE PATENTANMELDUNG

(21) Anmeldenummer: **82100038.7**

(22) Anmeldetag: **07.01.82**

(51) Int. Cl.³: **C 07 H 15/22**
A 61 K 31/70
//C07D317/24, C07D317/28,
C07D317/32, C07D317/20,
C07D319/06, C07D321/06

(30) Priorität: **17.01.81 DE 3101376**

(43) Veröffentlichungstag der Anmeldung:
**28.07.82 Patentblatt 82/30**

(84) Benannte Vertragsstaaten:
**AT BE CH DE FR GB IT LI NL SE**

(71) Anmelder: **BAYER AG**
**Zentralbereich Patente, Marken und Lizenzen**
**D-5090 Leverkusen 1, Bayerwerk(DE)**

(72) Erfinder: **Petersen, Uwe, Dr.**
**Auf dem Forst 4**
**D-5090 Leverkusen 1(DE)**

(72) Erfinder: **Stadler, Peter, Dr.**
**Am Ideck 8**
**D-5657 Haan(DE)**

(72) Erfinder: **Lockhoff, Oswald, Dr.**
**Bamberger Strasse 3**
**D-5090 Leverkusen 1(DE)**

(72) Erfinder: **Zeiler, Hans-Joachim, Dr.**
**Windrather Strasse 188**
**D-5620 Velbert 15(DE)**

(72) Erfinder: **Metzger, Karl Georg, Dr.**
**Pahlkestrasse 15**
**D-5600 Wuppertal 1(DE)**

## (54) Sisomicin-Derivate, Verfahren zu ihrer Herstellung und ihre Verwendung als Arzneimittel.

(57) 1-N-(Alkyloxycarbonyl)-sisomicinderivate, die in der Alkylgruppe (welche gegebenenfalls verzweigt sein kann) 2 bis 5 Hydroxylgruppen sowie gegebenenfalls eine Aminogruppe aufweisen, welche gegebenenfalls mit einer Alkyl-, Hydroxyalkyl- oder Aralkylgruppe substituiert sein kann.

EP 0 056 575 A1

0056575

BAYER AKTIENGESELLSCHAFT 5090 Leverkusen, Bayerwerk

Zentralbereich
Patente, Marken und Lizenzen Sft/Kü-c 16. Jan. 1981

## Sisomicin-Derivate, Verfahren zu ihrer Herstellung und ihre Verwendung als Arzneimittel

Die vorliegende Erfindung betrifft neue Sisomicin-Derivate, Verfahren zu ihrer Herstellung sowie ihre Verwendung als Arzneimittel.

Sisomicin ist eine antibakteriell wirksame Verbindung aus der Gruppe der Aminoglykosid-Antibiotika. Aminoglykosid-Antibiotika haben große Bedeutung bei der Bekämpfung bakterieller Infektionen gewonnen. Das Auftreten resistenter Keime mindert jedoch in vielen Fällen ihre breite Anwendbarkeit; des weiteren können Nebenwirkungen wie Oto- und Nephrotoxizität auftreten. Durch Derivatisierung gelingt es in einigen Fällen, diese Nachteile zu beseitigen.

So sind z.B. aus der Deutschen Offenlegungsschrift 2 753 769 1-N-(Aminoalkyloxycarbonyl)-sisomicin-Derivate bekannt geworden, die sich durch eine hohe Wirksamkeit gegenüber sisomicinresistenten Keimen auszeichnen.

In Weiterbildung der technischen Lehre von DE-OS 2 753 769 wurden nunmehr die antibiotisch besonders wirksamen erfindungsgemäßen Verbindungen gefunden.

Diese entsprechen der allgemeinen Formel I:

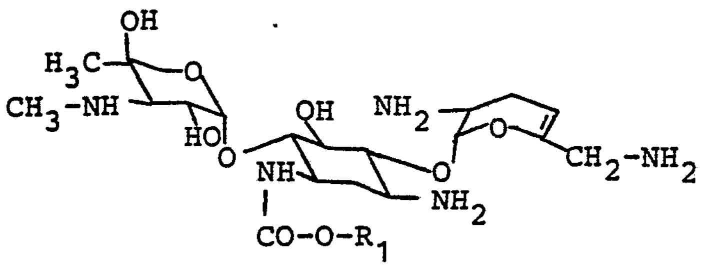

(I)

worin

$R_1$ einen Rest der Formel -A-XH bedeutet, wobei

A für einen gegebenenfalls verzweigten Alkylen- oder Alkenylrest mit 3 bis 7, vorzugsweise 4 bis 6 C-Atomen steht, welcher durch 2 bis 4 Hydroxylgruppen substituiert ist,

X für Sauerstoff oder vorzugsweise für eine Gruppe $-NR_2-$ steht und

$R_2$ Wasserstoff, eine Alkyl- oder Hydroxyalkylgruppe mit 1 bis 6, vorzugsweise 2 bis 4, C-Atomen oder eine Aralkylgruppe mit 7 bis 10 C-Atomen, vorzugsweise einen Benzylrest, darstellt.

Die erfindungsgemäßen Verbindungen zeichnen sich im Vergleich zu den bisher bekannten 1-N-(Aminoalkyloxy-

carbonyl)-sisomicin-Derivaten durch eine wesentlich verbesserte Verträglichkeit aus, ohne daß die hohe antibiotische Wirksamkeit eingeschränkt ist. Die neuen Verbindungen stellen somit eine Bereicherung der Medizin dar.

Die pharmazeutisch verwendbaren Salze leiten sich insbesondere von anorganischen oder organischen Säuren wie Schwefelsäure, Phosphorsäure, Salpetersäure, Salzsäure, Bromwasserstoffsäure, Essigsäure, Propionsäure, Ascorbinsäure, Zitronensäure usw. ab.

Geeignete Reste $R_1$ sind beispielsweise gerad- oder verzweigtkettige Polyhydroxyalkyl- und Polyhydroxyalkylaminoreste wie
2,3,4-Trihydroxybutyl,
4-Amino-2,3-dihydroxybutyl,
4-Benzylamino-2,3-dihydroxybutyl,
4-(2,3-Dihydroxypropylamino)-2,3-dihydroxybutyl,
4-Dimethylamino-2,3-dihydroxybutyl,
3-Amino-2,4-dihydroxybutyl,
2-Amino-3,4-dihydroxybutyl,
2,3,4,5-Tetrahydroxypentyl,
5-Amino-2,3,4-trihydroxypentyl,
5-Amino-2,3-dihydroxypentyl,
2,3,4,5,6-Pentahydroxyhexyl,
6-Amino-2,3,4,5-Tetrahydroxyhexyl,
6-Amino-4,5-dihydroxyhexyl,
1,4-Dihydroxy-3-amino-but-2-yl,

1,4-Dihydroxy-3-methylamino-but-2-yl,
1,4-Dihydroxy-3-propylamino-but-2-yl,
1,4-Dihydroxy-3-butylamino-but-2-yl,
1,4-Dihydroxy-3-benzylamino-but-2-yl,
1,4-Dihydroxy-3-(2-hydroxyethylamino)-but-2-yl,
2-Amino-3-hydroxy-2-hydroxymethyl-propyl,
3-Amino-2,2-bis-(hydroxymethyl)-propyl,
4-Amino-2,3-bis-(hydroxymethyl)-butyl.

Besonders bevorzugte Reste $R_1$ sind solche der Formeln

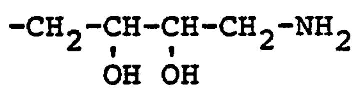

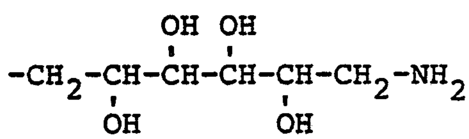

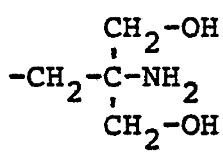

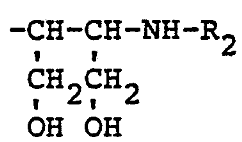

$R_2$=H, $C_3H_7$, $C_4H_9$, $CH_2-C_6H_5$, $CH_2CH_2-OH$

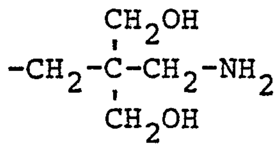

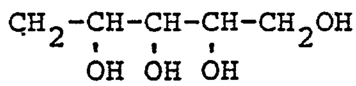

Die vorstehend aufgeführten Reste sind lediglich beispielhaft zu verstehen. Sie enthalten in der Regel chirale C-Atome und liegen als optisch reine Diastereomere oder Diastereomerengemische vor. Es kann von Vorteil sein, die erfindungsgemäßen Verbindungen als optisch reine Produkte zu verwenden.

Es wurde weiterhin gefunden, daß man die neuen Verbindungen der Formel I erhält, wenn man eine Verbindung der Formel II

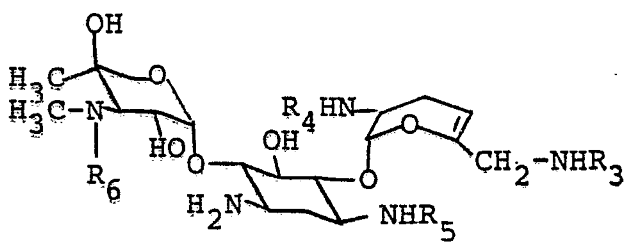

(II)

worin

$R_3$, $R_4$ und $R_5$ für $-CO-R_7$ oder $-S-R_8$ und

$R_6$ für $-S-R_8$ stehen,

wobei $R_7$ einen Rest der Formeln

$-CHal_3$, $-(CH_2)_{n_1}-B$, $-O-E$,

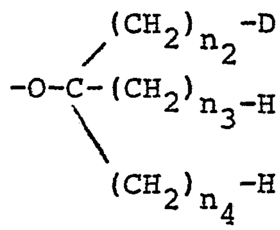

oder

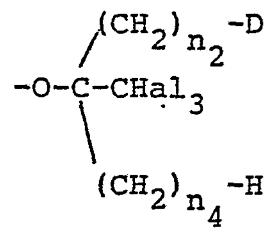

B und D Wasserstoff oder gegebenenfalls substituiertes Phenyl

E gegebenenfalls substituiertes Phenyl,

$n_1$ eine Zahl von 0 - 5,

$n_2$, $n_3$ und $n_4$ eine Zahl von 0 - 5,

Hal Fluor, Chlor oder Brom und

$R_8$ gegebenenfalls substituiertes Phenyl, Di- oder Triphenylmethyl

bedeuten,

in an sich bekannter Weise mit einem Acylierungsmittel der Formel III

G-CO-O-R' (III)

worin

G für eine Abgangsgruppe, vorzugsweise für Halogen wie Chlor und Brom, Azido oder gegebenenfalls substituiertes Phenoxy oder einen Rest der Formel

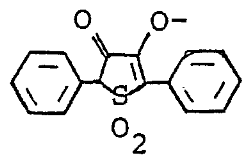

steht und R' für einen Rest $R_1$ steht, in dem gegebenenfalls vorhandene Aminogruppen durch geeignete Reste wie z.B. $-CO-R_7$ oder $-S-R_8$ und Hydroxylgruppen durch geeignete Schutzgruppen wie die Tritylgruppe, den Tetrahydropyranylrest oder durch Alkylidengruppen (wie beispielsweise Isopropyliden, Isobutyliden, Cyclohexyliden oder Benzyliden), die gleichzeitig zwei Hydroxylgruppen blockieren, geschützt sind, umsetzt, die Schutzgruppen $R_3$, $R_4$, $R_5$, $R_6$, sowie gegebenenfalls Tetrahydropyranylreste oder Alkylidengruppen, in üblicher Weise abspaltet und die resultierenden Verbindungen gegebenenfalls in ihre pharmazeutisch verwendbaren Salze überführt.

Unter gegebenenfalls substituiertem Phenoxy ist in diesem Zusammenhang insbesondere 4-Nitrophenoxy, Phenoxy und 2,4,5-Trichlorphenoxy zu verstehen.

Geeignete Substituenten der gegebenenfalls substituierten Phenyl-, Di- oder Triphenylmethylreste $R_8$ sind zum Beispiel 1 bis 3 Substituenten aus der Reihe Trifluormethyl, Nitro, $C_1$-$C_4$-Alkyl, $C_1$-$C_4$-Alkoxy, $C_1$-$C_4$-Alkoxycarbonyl oder Phenyl oder 1 bis 5 Halogenatome, vorzugsweise Chloratome. Als Beispiele für $-SR_8$-Gruppen seien o-Nitrophenylsulphenyl und 2,3,5-Trichlorphenylsulphenyl genannt.

Geeignete Substituenten der gegebenenfalls substituierten Phenylreste B, D und E sind 1 oder 2 Substituenten aus der Reihe Nitro, Halogen (vorzugsweise Chlor), $C_1$-$C_4$-Alkyl, $C_1$-$C_4$-Alkoxy und Phenyl.

Als Ausgangsstoffe der Formel (II) dienen bevorzugt 2',3,3",6'-Tetra-N-(o-nitrophenylsulfenyl)-sisomicin, 3"-N-(o-Nitrophenylsulfenyl)-2',3,6'-tris-N-trichloracetyl-sisomicin, 3"-N-(o-Nitrophenylsulfenyl)-2',3,6'-tris-N-trifluoracetyl-sisomicin, 3"-N-(o-Nitrophenylsulfenyl)-2',3,6'-tris-N-(2,2,2-trichlorethoxycarbonyl)-sisomicin, 3"-N-(o-Nitrophenylsulfenyl)-2',3,6'-tris-N-(1,1-dimethyl-2,2,2-trichlorethoxycarbonyl)-sisomicin, 3"-N-(o-Nitrophenylsulfenyl)-2',3,6'-tris-N-(4-methoxybenzyloxycarbonyl)-sisomicin, 3"-N-(o-Nitrophenylsulfenyl)-2', 3,6'-tris-N-phenoxycarbonylsisomicin und 3"-N-(o-Nitrophenylsulfenyl)-2',3,6'-tris-N-(tert.-butoxycarbonyl)-sisomicin, deren Herstellung nach den in DE-OS 2 726 197 beziehungsweise DE-OS 2 840 907 beschriebenen Verfahren erfolgt:

Die als Acylierungsmittel verwendeten Verbindungen der Formel (III) lassen sich bei Anwendung einer geeigneten Schutzgruppentechnik nach verschiedenen an sich bekannten Verfahren darstellen:

a) In einem Polyhydroxyalkyldicarbonsäureester wie Schleimsäurediethylester (1) (J.Org. Chem. 18, 952 /1953/) im Formelschema a) werden die Hydroxylgruppen durch Umsetzung mit einem Acetalisierungsreagens reversibel blockiert und anschließend die Estergruppierungen mit einem Reduktionsmittel wie Lithiumaluminiumhydrid zu freien primären Hydroxylgruppen reduziert.

Eine der freien Hydroxylgruppen wird mit einem Sulfonsäurechlorid in Pyridin in den Sulfonsäureester überführt, der sich durch eine nukleophile Substitutionsreaktion in ein Azid überführen läßt. Die folgende Reduktion liefert eine Aminoverbindung, beispielsweise (5), die nach Blockierung mit einer Schutzgruppe an der noch freien alkoholischen Funktion in ein reaktives Carbonat überführt wird. Im Formelschema a) ist eine Reaktionsfolge beispielhaft für den Schleimsäurediethylester (1) dargestellt, der zum aktivierten Carbonat (6) führt. Sie läßt sich in analoger Weise auch mit anderen Zuckersäureestern, Bis-hydroxymethylmalonsäureestern, sowie L-, D-, DL und meso-Weinsäureestern durchführen. Im Formelschema a) ist auch angegeben, daß man beipielsweise durch Umsetzung des Sulfonsäureesters (6) mit Benzylamin oder durch eine reduktive Alkylierung des Amins (5) mit Benzaldehyd zu einem substituierten Benzylamin gelangt, das nach Umsetzung mit 2-Nitrophenylsulfenylchlorid und Chlorkohlensäure-(4-nitrophenyl)-ester das aktivierte Carbonat (7) liefert. An Stelle von Benzylamin beziehungsweise Benzaldehyd lassen sich auch andere Amine oder Aldehyde für diese Substitutionsreaktion einsetzen.

Anmerkung:

In den nachfolgenden Strukturformeln sowie in den Strukturformeln der Beispiele werden die folgenden Abkürzungen verwendet:

NPS : 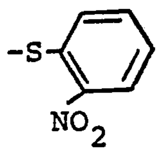

NP : 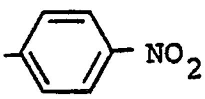

Tos : 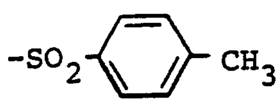

THP: 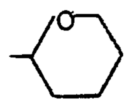

2-MBT: 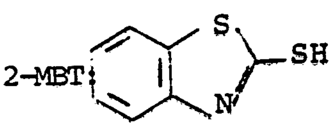

Formelschema a):

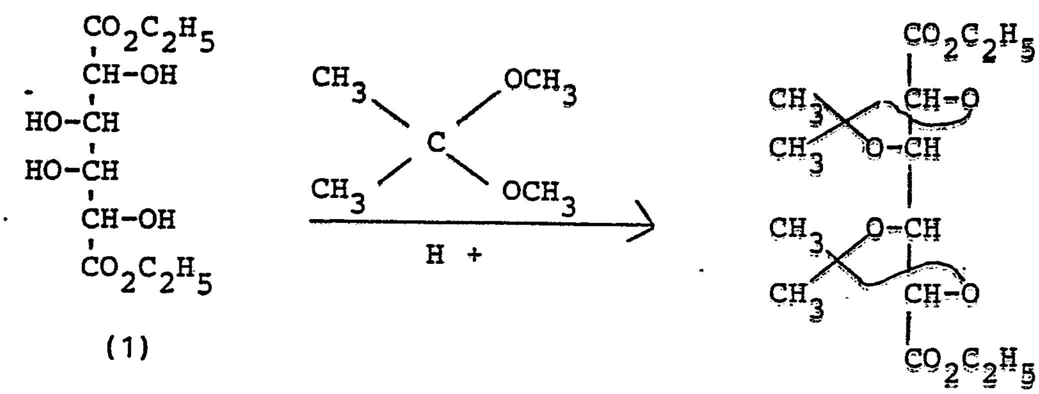


(1)

(2)

Reduktion

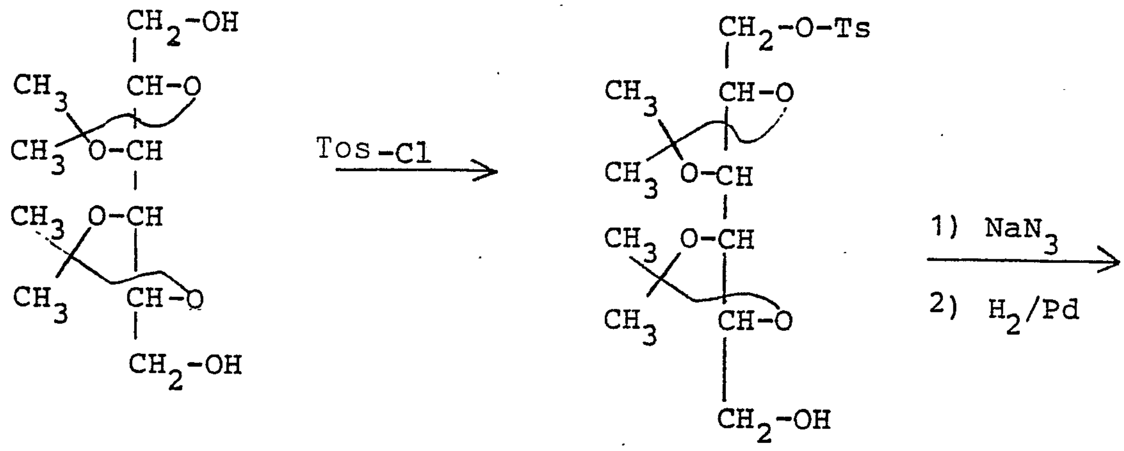
Tos-Cl
1) NaN$_3$
2) H$_2$/Pd

(3) (4)

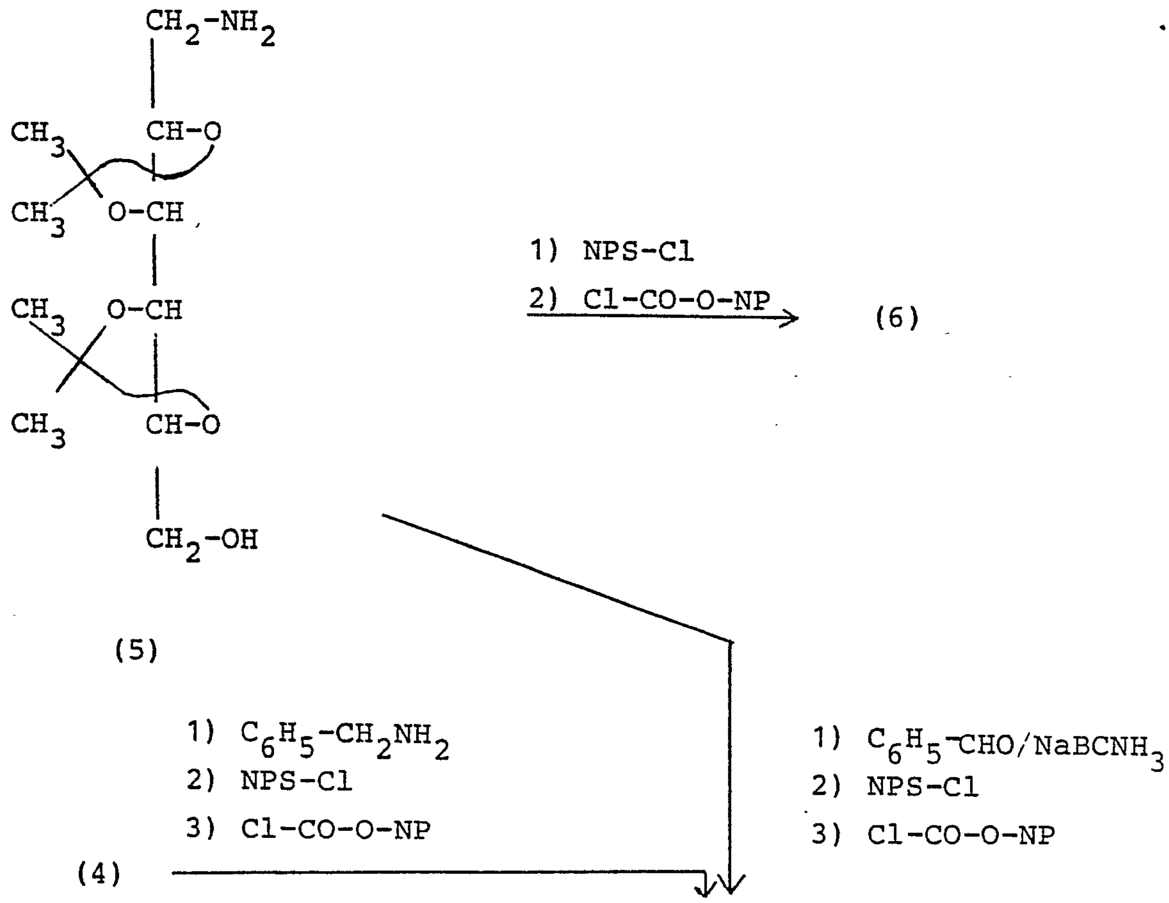
1) NPS-Cl
2) Cl-CO-O-NP
1) C$_6$H$_5$-CH$_2$NH$_2$
2) NPS-Cl
3) Cl-CO-O-NP
1) C$_6$H$_5$-CHO/NaBCNH$_3$
2) NPS-Cl
3) Cl-CO-O-NP

(5) (6) (4) (7)

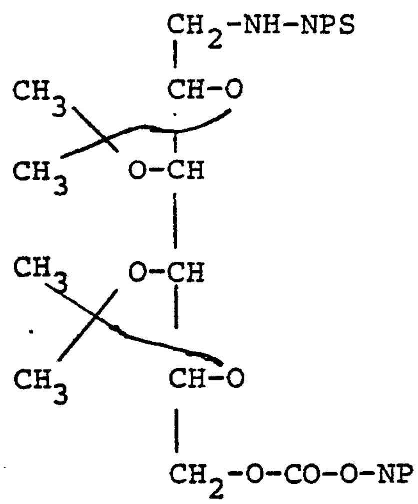 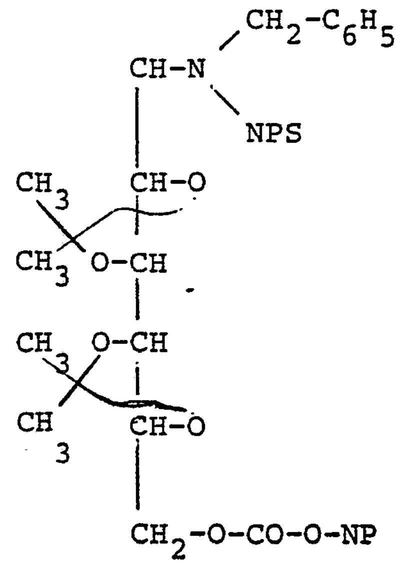

(6) (7)

b) Durch Umsetzung eines Polyhydroxyepoxids, dessen Hydroxylgruppen reversibel blockiert sind, mit Ammoniak oder einer Aminoverbindung erhält man eine Hydroxyaminoverbindung, die nach Einführung einer Aminoschutzgruppe und Überführung der freien Hydroxylgruppe in ein reaktives Carbonat zu dem gewünschten Acylierungsmittel (III) umgesetzt wird. Im Formelschema b) ist beispielhaft eine Reaktionsfolge ausgehend von 4,4-Dimethyl-3,5,8-trioxabicyclo [5.1.0]octan (8) (J. Org.Chem. 41, 2471 [1976]) aufgeführt.

Formelschema b):

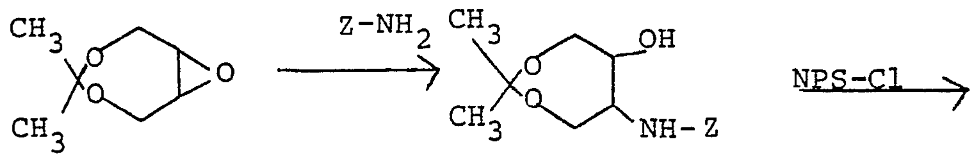


(8) (9)

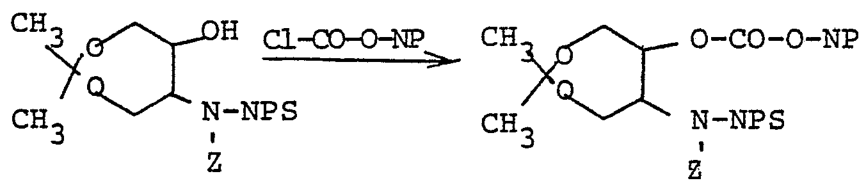


(10) (11)

c) In einer Polyhydroxyaminoverbindung wird die Aminogruppe acyliert und anschließend alle Hydroxygruppen bis auf eine reversibel blockiert. Nach Austausch der N-Acylschutzgruppe gegen eine N-(2-Nitrophenylsulfenyl)-schutzgruppe wird die freie Hydroxygruppe in ein reaktives Carbonat überführt. Formelschema c) zeigt beispielhaft die Umsetzungen ausgehend von 2-Amino-2-hydroxymethyl-1,3-propandiol.

Formelschema c):

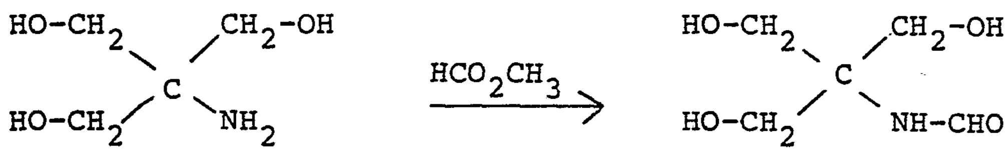


(12)

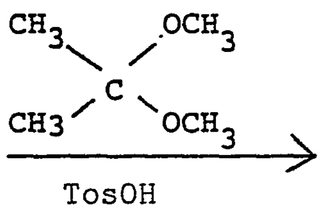


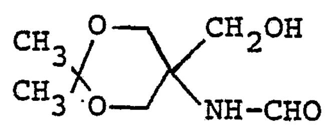

(13)

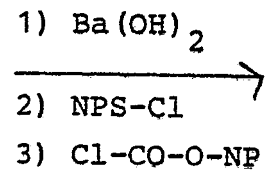


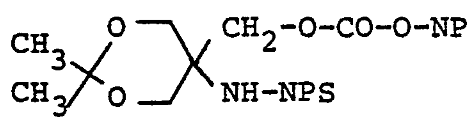

(14)

d) In Polyhydroxyverbindungen werden alle Hydroxylgruppen bis auf eine durch Acetalisierung reversibel blockiert und die verbliebene freie Hydroxylgruppe zu einem reaktiven Carbonat umgesetzt. Im Formelschema d) ist beispielhaft die Umsetzung von 2,3-4,5-Dibenzyliden-xylitol (15) mit 4,6-Diphenylthieno [3,4-d][1,3]dioxol-2-an-5,5-dioxid (16) (Angew. Chem. 88, 480 [1976]) zum aktivierten Carbonat (17) aufgeführt.

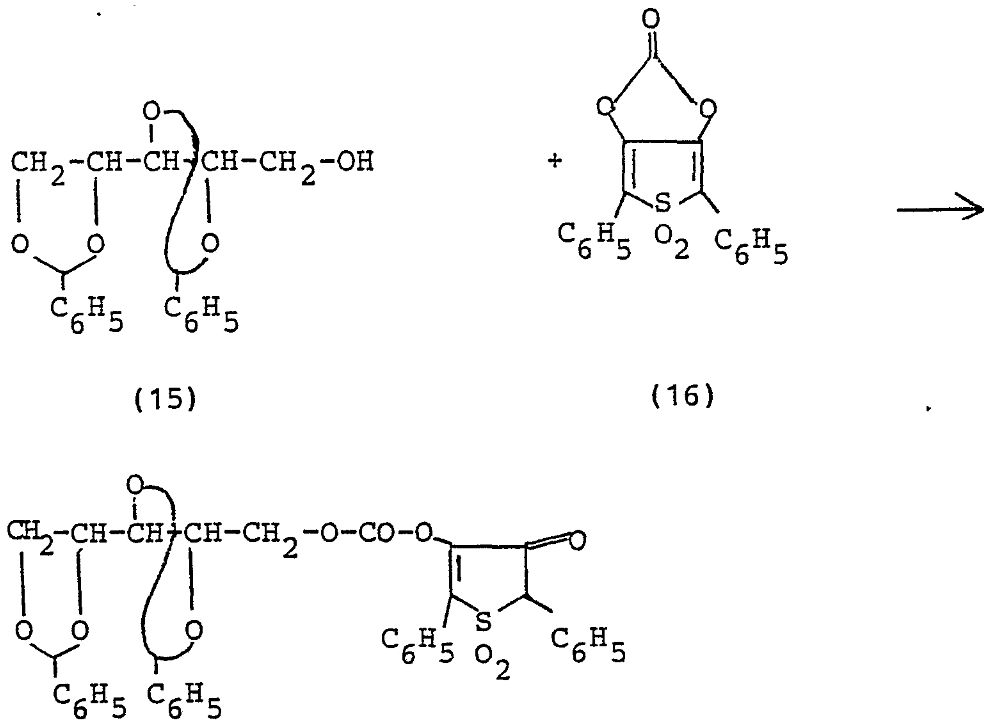

(15) (16)

(17)

Außer den in den Formelschemata a)-d) genannten Verbindungen (6), (7), (11), (14) und (17) sind weitere geeignete Acylierungsmittel (III) beispielsweise die folgenden Verbindungen:

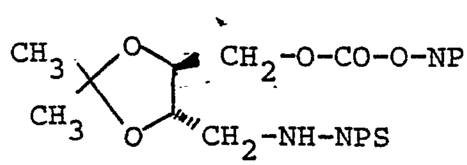

(18)

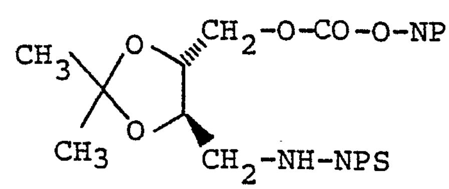

(19)

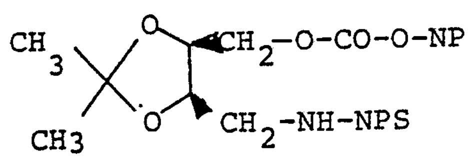

(20)

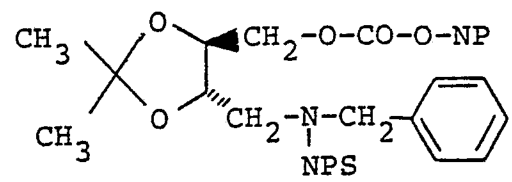

(21)

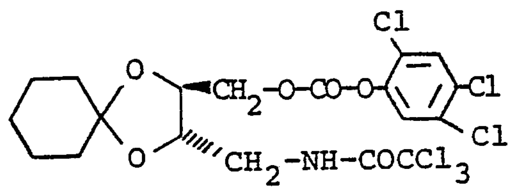

(22)

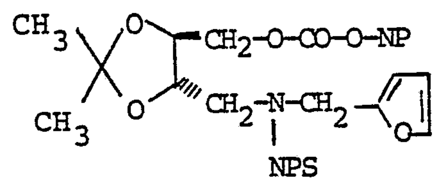

(23)

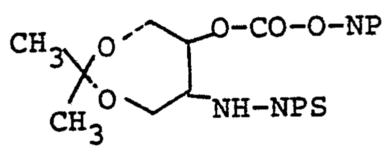

(24)

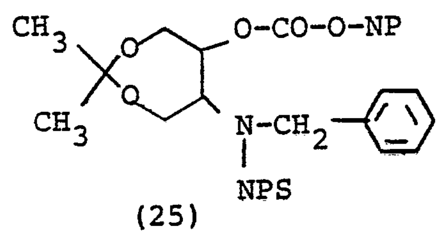

(25)

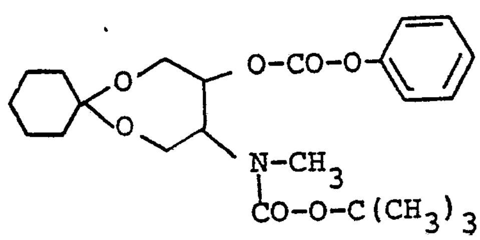

(26)

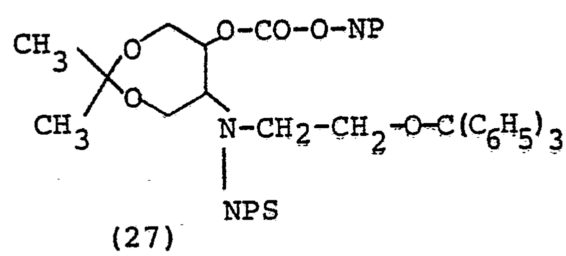

(27)

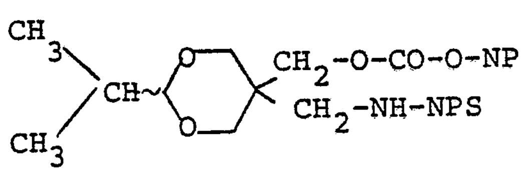

(28)

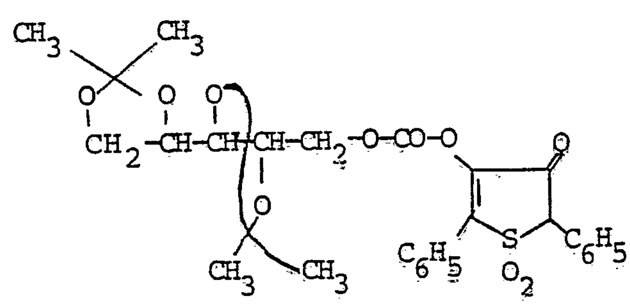

(29)

Bei der Herstellung der erfindungsgemäßen Verbindungen setzt man 1 Mol der Verbindung der Formel II mit etwa 1 bis 3 Mol, vorzugsweise 1,1 bis 1,5 Mol einer Verbindung der Formel III um, wobei alle inerten organischen Lösungsmittel wie Toluol, Chloroform, Methylenchlorid, Dimethylformamid, Dimethylacetamid, Dimethylsulfoxid, Äther wie Diethyläther, Dioxan und Tetrahydrofuran, Pyridin, Alkohole wie Methanol und Ethanol sowie deren Gemische als Verdünnungsmittel in Frage kommen.

Werden Säurebinder benötigt, so können alle üblichen organischen und anorganischen Säurebindungsmittel verwendet werden. Hierzu gehören vorzugsweise Alkali- und Erdalkalihydroxide wie Natriumhydroxid, Kaliumhydroxid oder Calciumhydroxid, Alkali- und Erdalkalicarbonate und -hydrogencarbonate wie Natriumcarbonat, Kaliumcarbonat, Natriumhydrogencarbonat und Calciumcarbonat, Calciumoxid, tertiäre aliphatische und aromatische Amine wie Triethylamin und N,N-Dimethylanilin sowie heterocyclische Basen wie Pyridin und Chinolin.

Die Reaktionstemperaturen können in einem großen Bereich variiert werden. Im allgemeinen arbeitet man bei Temperaturen von etwa -30°C bis +80°C, vorzugsweise zwischen etwa 0°C und etwa +40°C.

Die Umsetzung kann bei Normaldruck, aber auch bei erhöhtem Druck durchgeführt werden. Im allgemeinen arbeitet man bei Normaldruck.

Nach beendeter Umsetzung der Verbindungen II mit III werden im Molekül enthaltenen Schutzgruppen entfernt.

Die Abspaltung der Sulfenylschutzgruppen kann mit schwachen Säuren oder mit schwefelhaltigen, nukleophilen Reagentien wie beispielsweise $H_2S$, Thiophenol oder 2-Mercaptobenzthiazol erfolgen, die Abspaltung der übrigen Schutzgruppen kann mit wäßrigem Alkali- oder Erdalkalihydroxid oder mit Säuren wie Trifluoressigsäure, Perchlorsäure oder Bortrifluoridätherat erfolgen.

Verwendet man 3,2',6',3"-Tetra-N-(o-nitrophenylsulfenyl)-sisomicin und eine Verbindung der Struktur (6) als Ausgangsstoffe, so kann der Reaktionsablauf durch das folgende Formelschema wiedergegeben werden.

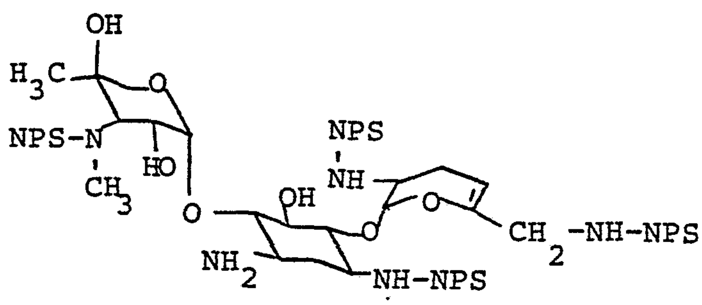

(30)

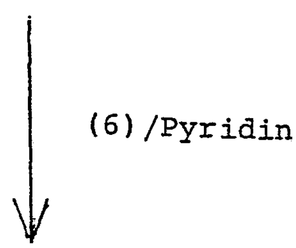

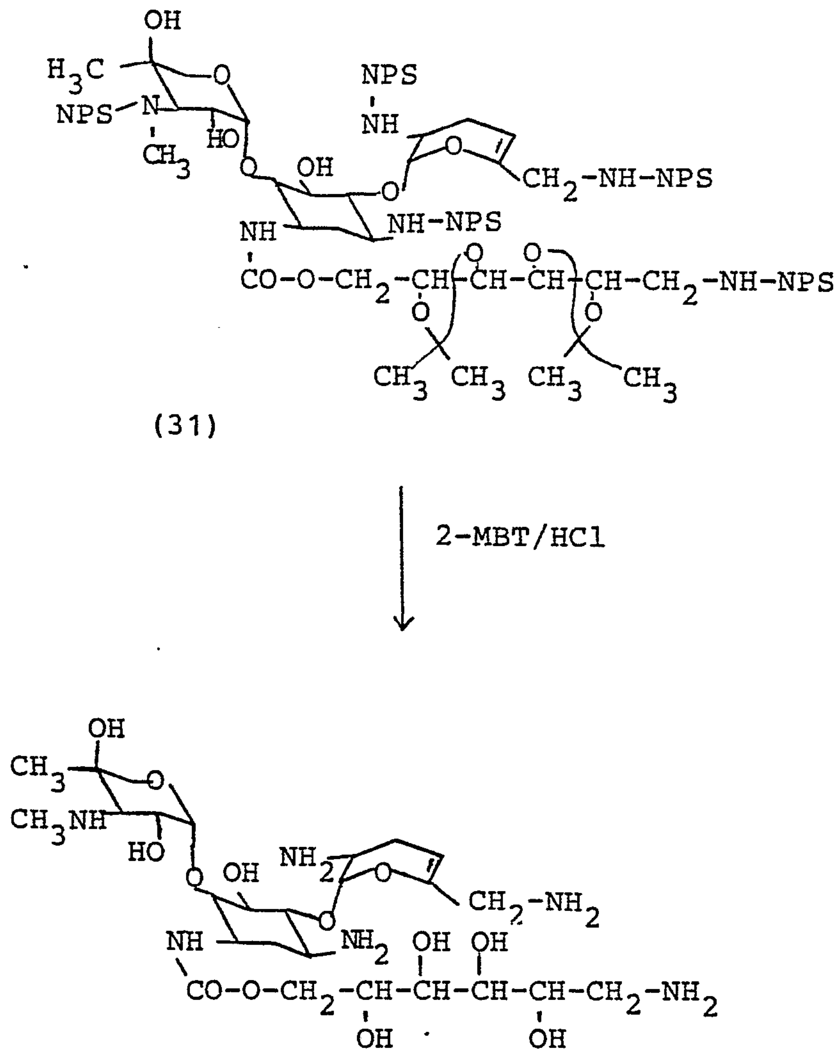


(31)

(32)

Als Beispiele für erfindungsgemäße Wirkstoffe seien genannt:

1-N-[(S,S)-, 1-N-[(R,R)-, 1-N-[(R,S)-, 1-N-[(S,R)-4-Amino-2,3-dihydroxybutyloxycarbonyl]-sisomicin

1-N-(3-Amino-2,4-dihydroxybutyloxycarbonyl)-sisomicin

1-N-(2- " -3,4- " )- "

1-N[(S,R)-, 1-N-[(R,S)-, 1-N-[(R,R)-, 1-N-[(S,S)-2,3,4-Trihydroxybutyloxycarbonyl]-sisomicin

1-N-(3-Amino-1,4-dihydroxybut-2-yloxycarbonyl)-sisomicin

1-N-(3-Methylamino-1,4-dihydroxybut-2-yloxycarbonyl)-sisomicin

1-N-(3-Propylamino-1,4- " -2- " )-sisomicin

1-N-(3-Benzylamino-1,4- " -2- " )-sisomicin

1-N-[3-(2-Hydroxyethylamino)-1,4- dihydroxybut-2-yloxycarbonyl]-sisomicin

1-N-[2-Amino-2,2-bis-(hydroxymethyl)-ethyloxycarbonyl]-sisomicin

1-N-[(S,S,R)-, 1-N-[(R,R,S)-, 1-N-[(R,S,R)-, 1-N-[(S,R,S)-, 1-N-[(S,R,R)-, 1-N-[(R,S,S)-, 1-N-[(R,R,R)-, 1-N-[(S,S,S)-2,3,4,5-Tetrahydroxypentyloxycarbonyl)]-sisomicin,

1-N-(5-Amino-2,3,4-trihydroxypentyloxycarbonyl)-sisomicin

1-N-[3-Amino-2,2-bis-(hydroxymethyl)-propyloxycarbonyl]-sisomicin

1-N-(2,3,4,5,6-Pentahydroxyhexyloxycarbonyl)-sisomicin

1-N-(6-Amino-2,3,4,5-tetrahydroxyhexyloxycarbonyl)-sisomicin

Die erfindungsgemäßen Verbindungen sind antimikrobielle Mittel mit einem breiten Wirkungsspektrum und besonderer Wirksamkeit gegen gram-negative Bakterien. Diese Eigenschaften ermöglichen ihre Verwendung als Arzneimittel, insbesondere bei der Bekämpfung von durch Bakterien hervorgerufenen Erkrankungen bei Mensch und Tier. Sie sind gut zur Prophylaxe und Chemotherapie von lokalen und systemischen Infektionen, insbesondere Infektionen des Urogenitalsystems, in der Human- und Tiermedizin geeignet, die durch gram-negative Bakterien, z.B. E.coli, Proteus, Klebsiella und Pseudomonas verursacht werden. Hemmhöfe im Agar-Lochtest wurden z.B. gegen folgende Bakterienstämme bei einer Konzentration von 100 Mikrogramm/1 ml gefunden:

| | | |
|---|---|---|
| Pseudomonas | aerug. | 5737 |
| Pseudomonas | aerug. | F 41 |
| Klebsiella | pneum. | 2 München |
| Klebsiella | pneum. | 1 Düsseldorf |
| E. coli | | Münster |
| E. coli | | Neumann |

Das Verfahren zur Behandlung von durch Bakterien hervorgerufenen Erkrankungen ist dadurch gekennzeichnet, daß man die neuen 1-N-Derivate Menschen und Tieren appliziert, die an diesen Erkrankungen leiden.

Im allgemeinen hängt die zu verabreichende Dosis der Verbindungen der Erfindung vom Alter und Gewicht des Lebewesens, von der Verabreichungsart, vom Typ und von der Schwere der bakteriellen Infektion ab. Die Dosierung der erfindungsgemäßen Verbindungen ist gewöhnlich der Dosierung der 1-N-unsubstituierten Verbindung ähnlich. Der Dosierungsspielraum beträgt von 20 mg/Tag/Mensch bis 2000/mg/Tag/Mensch, vorzugsweise 100 mg - 500 mg/Tag.

Die Verbindungen der Erfindung können oral verabreicht werden. Die Verabreichung kann in Einzelgaben oder auf mehrere Gaben verteilt erfolgen. Die Verbindungen können auch topisch verabreicht werden in der Form von Salben, Cremen oder Lotionen. Pharmazeutische Trägerstoffe, für diese Formulierungen umfassen Wasser, Öle, Fette, Polyester und Polyole.

Wie im folgenden gezeigt wird, ist das Verfahren zur Herstellung von Arzneimittel, welche die neuen 1-N-Sisomicin-Derivate enthalten, dadurch gekennzeichnet, daß man die neuen Verbindungen mit pharmazeutisch geeigneten Träger- und/oder Zusatzstoffen vermischt.

Zur oralen Verabreichung der Verbindungen dieser Erfindung können Tabletten, Kapseln oder Elixiere Anwendung finden, die Verbindungen können aber auch dem Tierfutter zugemischt werden.

Im allgemeinen enthalten topische Präparationen ca. 0,1 bis ca. 3,0 g der Verbindungen der Erfindung pro 100 g Salbe, Creme oder Lotion. Die topische Verabreichung erfolgt ca. 2 bis 5 mal am Tag.

Die antibakteriellen Mittel der Erfindung können in flüssiger Form als Lösungen oder Suspensionen zur

Anwendung an Ohren und Augen oder zur parenteralen Verabreichung in Form intramuskulärer oder intravenöser Injektionen vorliegen. Injektionslösungen oder -suspensionen werden gewöhnlich so verabreicht, daß ca. 1 bis 15 mg Wirkstoff pro Kilogramm Körpergewicht in 2 bis 4 Dosen pro Tag in den infizierten Organismus gelangen. Die genaue Dosis hängt von der Art der Infektionen, der Empfindlichkeit des infizierenden Keims und von den individuellen Charakteristika des zu Behandelnden ab.

Die nachfolgenden Beispiele erläutern die Herstellung der erfindungsgemäßen Verbindungen. Wenn nicht anders vermerkt, sind Mengenangaben als Gewichtsteile bzw. Gewichtsprozente zu verstehen.

Für die chromatographischen Trennungen in den Ausführungsbeispielen wurden die folgenden Laufmittelsysteme benutzt:

A. Toluol/Essigsäureethylester (2:1)
B. Methylenchlorid/Methanol (95:5)
C. Methylenchlorid/Methanol/20% wäßriges Ammoniak (2:4:1)
D. Methylenchlorid/Methanol/17% wäßriges Ammoniak (150:20:1)

Die analytischen Trennungen wurden auf Kieselgel-DC-Fertigplatten (Merck, Darmstadt) und die präparativen Trennungen mit Kieselgel 60 (Merck, Darmstadt) durchgeführt.

Beispiel 1

1-N-([S,S]-4-Amino-2,3-dihydroxy-butyloxycarbonyl)-sisomicin (39)

Reaktionsschema:

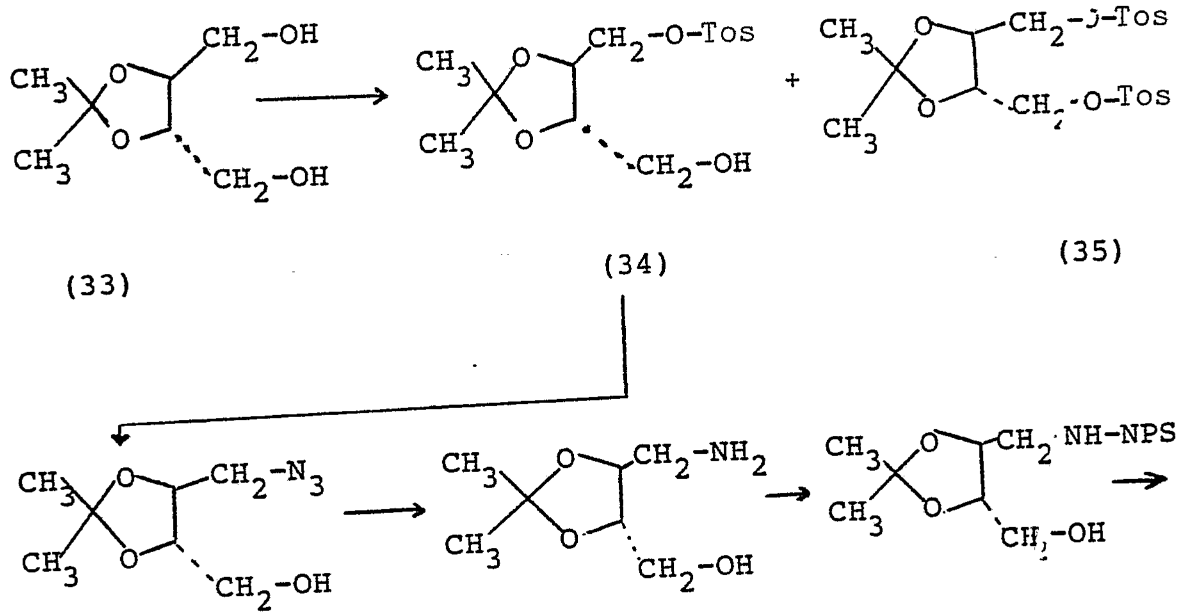

(33) (34) (35)

(18)

(36) (37) (38)

(30)

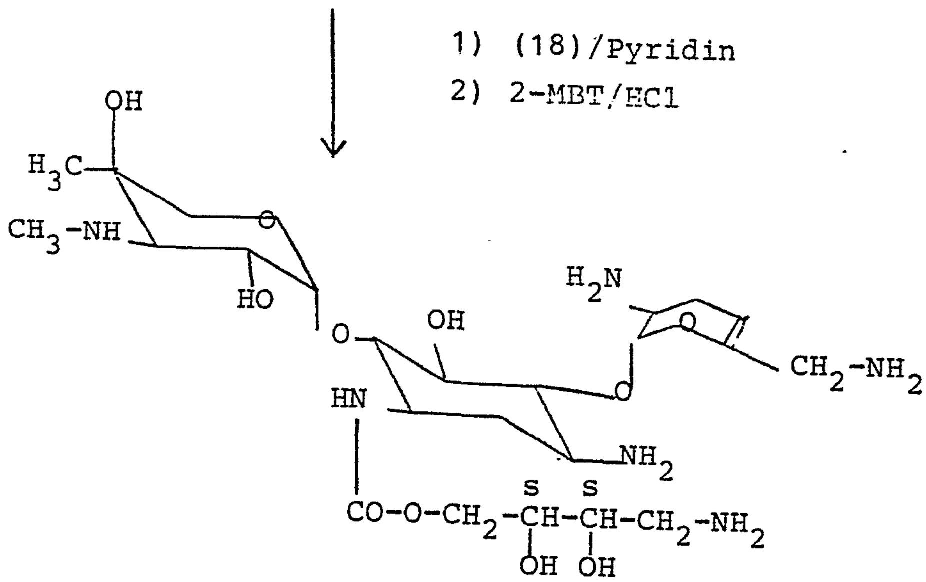


(39)

a) Herstellung der Verbindungen (34) und (35)

Eine Lösung von 162 g (1 mol) (+)-trans-4,5-Bis /hydroxymethyl/-2,2-dimethyl-1,3-dioxolan (Synthesis 1979, 350) in 600 ml Pyridin versetzt man bei -15°C portionsweise mit 190,5 g p-Toluolsulfonsäurechlorid (1 mol) und rührt, bis die Mischung homogen ist. Die Mischung wird über Nacht auf -25° gehalten, im Hochvakuum eingeengt, mit System A verrührt und vom Ungelösten abfiltriert. Die Mutterlauge chromatographiert man zur Auftrennung der Komponenten (34) und (35) an 1,4 kg Kieselgel (System A). Ausbeute:
Verbindung (34):176 g farbloses Oel (55,7%);
Rf-Wert (System A): 0,29; NMR: 1 Tosylrest
Verbindung (35): 53 g weißes Kristallisat (11,3%);
Schmp. 88-91°; Rf-Wert (System A): 0,78, NMR:
2 Tosylreste.

b) Herstellung der Verbindung (36)

Man erhitzt eine Mischung von 162 g (34) (0,51 mol) in 750 ml Dimethylformamid und 77 g $NaN_3$ (1,18 mol) in 77 ml Wasser 1/2 Tag unter Rückfluß, engt im Hochvakuum ein, verrührt mit wenig System A, saugt vom Niederschlag ab und filtriert die Mutterlauge über 150 g Kieselgel (Elutionsmittel: System A).

Ausbeute: 68,9 g (72%) Oel; IR: 2090/cm; Rf-Wert (System A): 0,14.

c) Herstellung der Verbindung (37)

63,9 g (36) (0,34 mol) in 500 ml Essigsäureethylester werden mit 4 g Raney-Ni unter Durchleiten von Wasserstoff während 3 Tagen hydriert und die Reaktion dünnschichtchromatographisch verfolgt (System A). Man filtriert vom Katalysator ab und engt im Vakuum ein.

Ausbeute: 50 g (91%) farbloses Oel; Rf-Wert (System D): 0,29.

d) Herstellung der Verbindung (38)

Zu einer Lösung von 50 g (37) (0,31 mol) in 300 ml Dioxan/156 ml 2n-NaOH werden bei

pH > 8 gleichzeitig eine Lösung von 59 g 2-Nitrophenylsulfenylchlorid (NPS-Cl)(0,21 mol) /300 ml Dioxan und 150 ml 2n-NaOH getropft und 2 Stunden bei Raumtemperatur gerührt. Man engt im Vakuum ein, löst in 300 ml Methylenchlorid und versetzt mit 100 ml Wasser. Die abgetrennte wäßrige Phase wird mit 2 mal 50 ml Methylenchlorid extrahiert und die vereinigten organischen Phasen mit insgesamt 100 ml Wasser gewaschen, mit $Na_2SO_4$ getrocknet, im Vakuum eingeengt und über 200 g Kieselgel filtriert (Elutionsmittel: System A).

Ausbeute: 76 g (78%) oranges Oel; Rf-Wert (System A): 0,25.

e) Herstellung der Verbindung (18)

Eine Lösung von 76 g (38) (0,24 mol) in 450 ml Pyridin versetzt man mit 48,8 g Chlorameisensäure-p-nitrophenylester (0,24 mol) und rührt über Nacht bei Raumtemperatur. Wenn dünnschichtchromatographisch kein (38) mehr nachweisbar ist, wird im Hochvakuum eingeengt, in 200 ml Methylenchlorid aufgenommen, mit 3 mal 75 ml Wasser gewaschen und mit $Na_2SO_4$ getrocknet. Man engt ein und reinigt chromatographisch (System A) an 300 g Kieselgel.

Ausbeute: 81 g oranges Oel; DC (System A):
1 Hauptkomponente (Rf-Wert 0,64), 1 Nebenkomponente (Rf-Wert 0,50)(identisch mit p-Nitrophenol).

f) Herstellung der Verbindung (39)

Eine Lösung von 33 g ($3.10^{-2}$ mol) 3,2', 6', 3"-Tetra-N-(o-nitrophenylsulfenyl)-sisomicin (30) (Deutsche Offenlegungsschrift 2726197) in 160 ml Pyridin wird mit 17,6 g (18) ($3,6 \times 10^{-2}$ mol) in 20 ml Methylenchlorid versetzt und 1 Tag bei Raumtemperatur gerührt. Man engt im Hochvakuum ein und isoliert das Acylierungszwischenprodukt durch Chromatographie an 800 g Kieselgel (System B; $R_f$-Wert: 0,45): 39,5 g oranges Festprodukt (als erstarrter Schaum). Die Abspaltung der NPS-Gruppen erfolgt durch Lösen in 70 ml Methylenchlorid, Versetzen mit einer Lösung von 85 g 2-Mercaptobenzthiazol in 300 ml Methanol/50 ml Methylenchlorid und Ansäuern mit 15 ml konz. HCl. Man extrahiert mit 2 x 100 ml Wasser, wäscht die wäßrige Phase mit 3 x 50 ml Methylenchlorid, dampft die saure wäßrige Lösung bis auf 100 ml ein und läßt sie zur Abspaltung der Isopropylidenschutzgruppe 7 Tage bei Raumtemperatur stehen. Danach wird die Lösung mit einem handelsüblichen Ionenaustauscher alkalisch gestellt, eingeengt und über 200 g Kieselgel chromatographiert und es werden folgende Fraktionen isoliert:

I. 3,5 g DC (System C): 1 Hauptkomponente ($R_f$-Wert 0,13), 1 Nebenkomponente ($R_f$-Wert 0,24) = Sisomicin

II. 6,9 g DC (System C): 1 Hauptprodukt
($R_f$-Wert 0,13),

III. 0.4 g DC (System C): 1 Hauptprodukt
($R_f$-Wert 0,13),
1 Spur ($R_f$-Wert 0,08)

Die Fraktion II enthält die reine Verbindung (39); in den Fraktionen I und III liegen neben (39) noch Verunreinigungen vor, die durch Rechromatographie entfernt werden.

Beispiel 2

1-N-(/R̄,R/-4-Amino-2,3-dihydroxy-butyloxycarbonyl)-sisomicin (40)

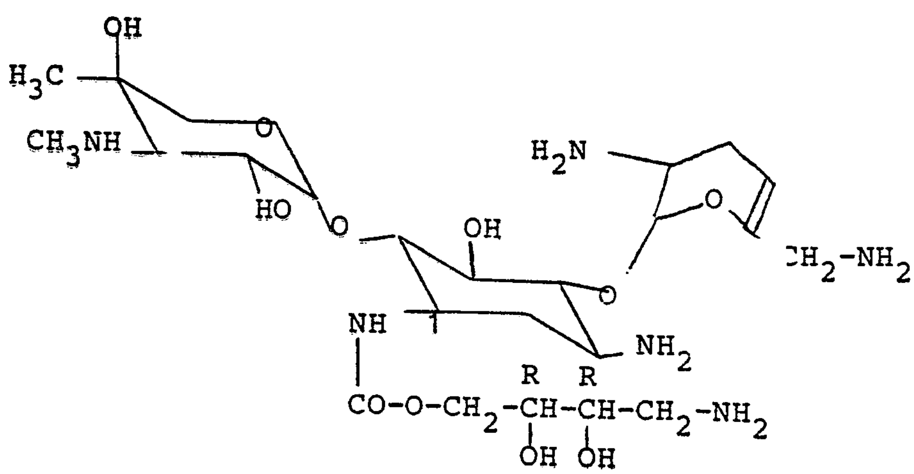

Die Herstellung erfolgt analog Beispiel 1 ausgehend von (-)-trans-4,5-Bis/hydroxymethyl/-2,2-dimethyl-1,3-dioxolan, das entsprechend Verbindung (33) nach

B.A. Murrer et al., Synthesis 1979, 350 aus D(-)-Weinsäurediethylester hergestellt wird. Die $R_f$-Werte sämtlicher Zwischenprodukte sind identisch mit denen der entsprechenden Isomeren, die in Beispiel 1 aufgeführt sind. Das $^{13}C$-NMR-Spektrum von (40) ist in Tabelle 1 aufgeführt:

Tabelle 1: $^{13}$C-Shifts $\delta$ (ppm, rel. zu TMS=0) für (40) in $D_2O$

| C-Atome, d.h. Zuordnung | Signallagen | rel. Intensitäten |
|---|---|---|
| C-1" | 100.441 | 3.772 |
| C-2" | 70.962 | 3.052 |
| C-3" | 64.239 | 3.193 |
| C-4" | 72.824 | 3.795 |
| C-5" | 69.390 | 2.429 |
| $N-CH_3$ an C-3" | 37.456 | 3.689 |
| $CH_3$ and C-4" | 22.388 | 4.200 |
| C-1 | 52.139 | 2.080 |
| C-2 | 32.225 | 2.101 |
| C-3 | 49.748 | 5.319 |
| C-4 | 81.618 | 1.736 |
| C-5 | 75.584 | 3.678 |
| C-6 | 84.105 | 2.072 |
| C-1' | 99.719 | 2.244 |
| C-2' | 47.229 | 4.758 |
| C-3' | 25.453 | 3.159 |
| C-4' | 98.836 | 3.621 |
| C-5' | 147.715 | 1.364 |
| C-6' | 43.362 | 2.885 |
| NH-CO-O- vom Rest an 1-N | 158.483 | 1.308 |
| $-OCH_2$ vom Rest an 1-N | 66.790 | 1.528 |
| $-CH_2-NH_2$ vom Rest an 1-N | 42.752 | 4.055 |
| -CH(OH)-CH(OH)- vom Rest an 1-N | 71.332 u. 68.427 | 1.899 u. 3.281 |

Beispiel 3

1-N-([R,S,R,S]-6-Amino-2,3,4,5-tetrahydroxy-hexyl-oxycarbonyl)-sisomicin (32)

Reaktionsschema (sh. Seite 10/11):

(1) → (2) → (3) → (4) +

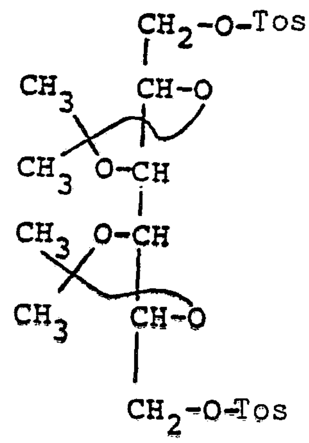

(41)

(4) → (5) → (6) —+ (30)→ (31) → (32)

a) Herstellung der Verbindung (2)

36 g Schleimsäurediethylester (1) (J.Org.Chem. 18, 952 [1953]) werden mit 29,8 g 2,2-Dimethoxypropan (0,29 mol) und 0,3 g p-Toluolsulfonsäure in 400 ml Aceton 13 Stunden unter Rückfluß erhitzt, wobei das Kondensat über 50 g Zeolith (4Å; bei 100°C/12 mm getrocknet) getrocknet wurde. Zur Aufarbeitung wurde mit 0,2 g $Na_2CO_3$ versetzt, filtriert, eingeengt und der Rückstand aus 50 ml Ethanol umkristallisiert.

Ausbeute: 25,2 g (53%); Schmp. 85-87°C.
IR: 1760 / cm; NMR ($CDCl_3$): $\delta$ = 1,3 t (2 Ester - $CH_3$), 1,47 d (6-Isopropyliden - $CH_3$), 4,25 q (2 Ester - $CH_2$), 4,5 m (4 OH)
$R_f$-Wert (System A) : 0,64

b) Herstellung der Verbindung (3)

Man legt bei 0°C eine Suspension von 5,7 g $LiAlH_4$ (0,15 mol) in 250 ml absol. Ether unter $N_2$ vor und tropft in 30 Minuten eine Lösung von 24,3 g (2) (0,07 mol) in 400 ml absol. Ether bei einer Innentemperatur von 0-15°C zu. Anschließend wird die Suspension 5 Stunden unter Rückfluß erhitzt und unter $N_2$ bei -10°C bis -5°C vorsichtig mit 15 ml Wasser und 15 ml 4n NaOH versetzt. Der Niederschlag wird abgesaugt, mit Ether gewaschen und noch mit 250 ml Dioxan 3 Stunden ausgekocht.

Die vereinigten Mutterlaugen engt man ein und kristallisiert aus 25 ml Isopropanol um.

Ausbeute: 6,3 g (29,4%), Schmp. 111-113°C; $R_f$-Wert (System A): 0,1. NMR ($CDCl_3$): $\delta$ = 1,4 breites s (4-Isopropyliden-$CH_3$), 2,62 s (2 OH; tauschen mit $CD_3OD$ aus), 3,82 m (2 $CH_2$-O), 4,0 m (4 CH-O).

c) Herstellung der Verbindungen (4) und (41)

6 g (3)(0,023 mol) werden in 15 ml absol. Pyridin gelöst und bei -15°C portionsweise mit 4,4 g p-Toluolsulfonsäurechlorid (0,023 mol) innerhalb von 5 Minuten versetzt. Man rührt bis zur homogenen Mischung bei -15°C nach, läßt über Nacht bei -25°C bis -30°C stehen und verrührt bei 0°C mit 100 ml Wasser. Der ausfallende Niederschlag wird abgesaugt, mit Wasser gewaschen und bei 40°C/12 mm getrocknet (7,5 g). Zur Trennung beider Komponenten chromatographiert man an 150 g Kieselgel mit $CH_2Cl_2$ / Methanol (99:1) als Laufmittel:

Verbindung (41): 2 g Schmp. 164-166°C; $R_f$-Wert 0,72; NMR zeigt 2 Tosylreste
Verbindung (4): 3,5 g, Schmp. 83-84°C; $R_f$-Wert: 0,29; NMR zeigt 1 Tosylrest.

d) Herstellung der Verbindung (5)

3,33 g (4) ($8,3 \times 10^{-3}$ mol) in 15 ml Dimethyl-

formamid versetzt man mit einer Lösung von 1,2 g $NaN_3$ (1,84 x $10^{-2}$mol) in 2 ml Wasser und erhitzt 9 Stunden zum Rückfluß. Man engt im Vakuum ein, verrührt den Rückstand mit etwas Toluol/Essigester (2:1), filtriert vom Salz ab und chromatographiert das Filtrat an 150 g Kieselgel mit System A als Laufmittel.

Ausbeute: 1,5 g Öl (62,2%); IR: 2100/cm; $R_f$-Wert (System A): 0,27.

1,5 g (5,2 x $10^{-3}$ mol) des so erhaltenen Öls werden in 80 ml Essigsäurethylester mit 1 g Pt-Mohr 2 Tage bei Raumtemperatur und Normaldruck hydriert und die Reaktion durch DC-Kontrolle verfolgt. Man filtriert den Katalysator ab, engt ein und erhält ein weißes Festprodukt, das nach DC (System A) kein Azid mehr enthält.

Ausbeute: 1,1 g (81%), Schmp. 130-133 °C.

e) Herstellung der Verbindung (6)

0,91 g (5) (3 x $10^{-3}$ mol) werden in 3,5 ml Dioxan und 1,75 ml 2 n NaOH vorgelegt und bei pH 8-9 gleichzeitig eine Lösung von 0,65 g NPS-Cl (3,5 x $10^{-3}$mol) in 3,5 ml Dioxan und 2,2 ml 2n-NaOH zugetropft. Nach 60 Minuten ist im DC (System A) eine neue Komponente nachweisbar, die man nach Abdampfen des Lösungsmittels durch

Filtration über 30 g Kieselgel mit System A sauber erhält.

Ausbeute: 0,8 g (64%), Schmp. 112-115°C;
$R_f$-Wert (System A): 0,2
NMR ($CDCl_3$):  = 1,38 2 d (4 Isopropyliden-$CH_3$), 2,22 t breit (OH), 3,23 m (3H), 3,78 m (6H), 7,0 - 8,4 m (4H, NPS-Rest).

Eine Lösung von 750 mg (1,8 x $10^{-3}$ mol) des so erhaltenen Produkts in 4 ml absol. Pyridin wird mit 360 mg Chlorameisensäure-p-nitrophenylester (1,8 x $10^{-3}$ mol) 2 Stunden bei Raumtemperatur verrührt, anschließend im Hochvakuum eingeengt und chromatrographisch an 30 g Kieselgel mit System A gereinigt.

Ausbeute: 0,9 g; $R_f$-Wert (System A): 0,54.

f) Herstellung der Verbindung (32)

Eine Lösung von 330 mg 3,2', 6', 3""-Tetra-N-(o-nitrophenylsulfenyl)-sisomicin (30) (3 x $10^{-4}$ mol) in 1,5 ml Pyridin wird mit 260 mg (6) versetzt und über Nacht bei Raumtemperatur stehengelassen. Nach Eindampfen der Lösung wird das Reaktionsprodukt (31) an 40 g Kieselgel (System B) isoliert und analog Beispiel 1, f gespalten.
Zur Abspaltung der beiden Isopropylidengruppen bleibt die saure wäßrige Lösung 11 Tage bei Raumtemperatur stehen, wobei die stufenweise Abspaltung über ein Mono-isopropyliden-Derivat ($R_f$-Wert: 0,34; System C) zum völlig deblockierten Derivat ($R_f$-Wert: 0,02; System C) sehr langsam erfolgt. Anschließend wird mit einem handelsüblichen Ionenaustauscher alkalisch gestellt, eingeengt und zur Reinigung an 5 g Kieselgel mit System C als Laufmittel chromatographiert.

Ausbeute: 47 mg (24 %)
$R_f$-Wert (System C): 0,02 (zum Vergleich: Sisomicin $R_f$ = 0,16)

Das $^{13}$C-Spektrum von (32) ist in Tabelle 2 aufgeführt:

Tabelle 2: $^{13}$C-Shifts (ppm, rel. zu TMS=0) für (32) in $D_2O$

| C-Atome, d.h. Zuordnung | Signallagen | rel. Intensitäten |
|---|---|---|
| C-1" | 101.271 | < 1 |
| C-2" | 70.272 | 2.262 |
| C-3" | 64.463 (s. unten) | 3.330 |
| C-4" | 72.487 | 3.097 |
| C-5" | 68.957 | 2.959 |
| $N-CH_3$ an C-3" | 37.135 | 4.049 |
| $CH_3$ an C-4" | 22.308 | 4.699 |
| C-1 | 52.139 | 2.090 |
| C-2 | 35.386 | 2.492 |
| C-3 | 49.764 | 7.372 |
| C-4 | 81.651 | < 1 |
| C-5 | 75.584 | 2.961 |
| C-6 | 83.688 | 1.863 |
| C-1' | 100.136 | 4.549 |
| C-2' | 47.133 | 4.413 |
| C-3' | 25.453 | 3.222 |
| C-4' | 99.590 | 2.792 |
| C-5' | 145.946 | < 1. (breit) |
| C-6' | 43.891 | 3.669 |
| CO vom Rest an N-1 | 158.579 | 1.907 |
| $CH_2NH_2$ vom Rest an N-1 | 42.415 | 4.160 |
| CHOH, $\alpha$ zu $CH_2$ vom Rest an N-1 | 71.127 | 3.092 |
| restl. CHOH vom Rest an an N-1 um | 68.3 | 4.936 (verbreitert) |
| $CH_2O$ vom Rest an N-1 | 64.463 (s. oben) | 3.330 |

Beispiel 4

1-N-(3-Amino-2,2-bis-/hydroxymethyl7-propyloxycarbonyl)-sisomicin (51)

Reaktionsschema:

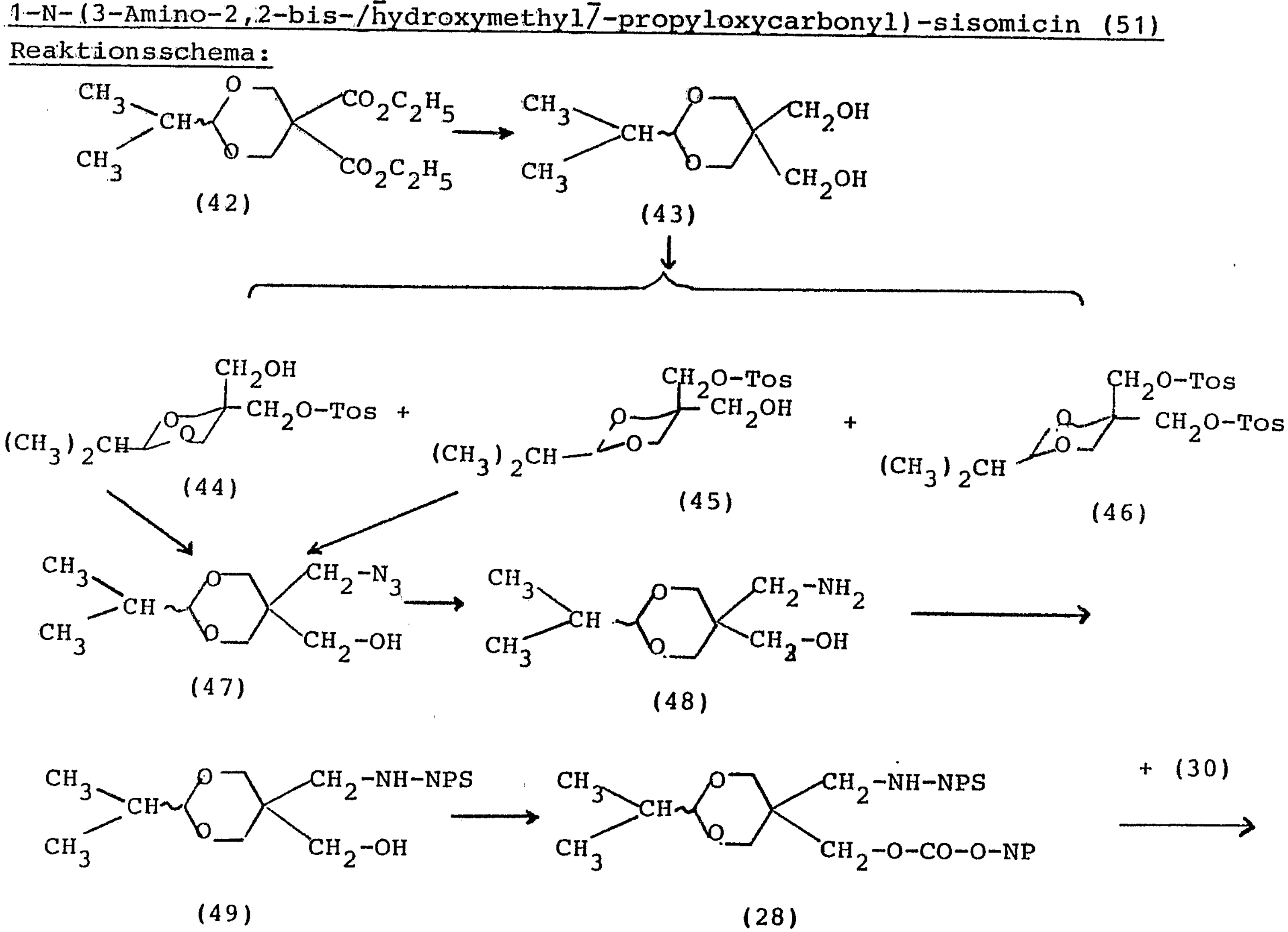


(42) (43) (44) (45) (46) (47) (48) (49) (28)

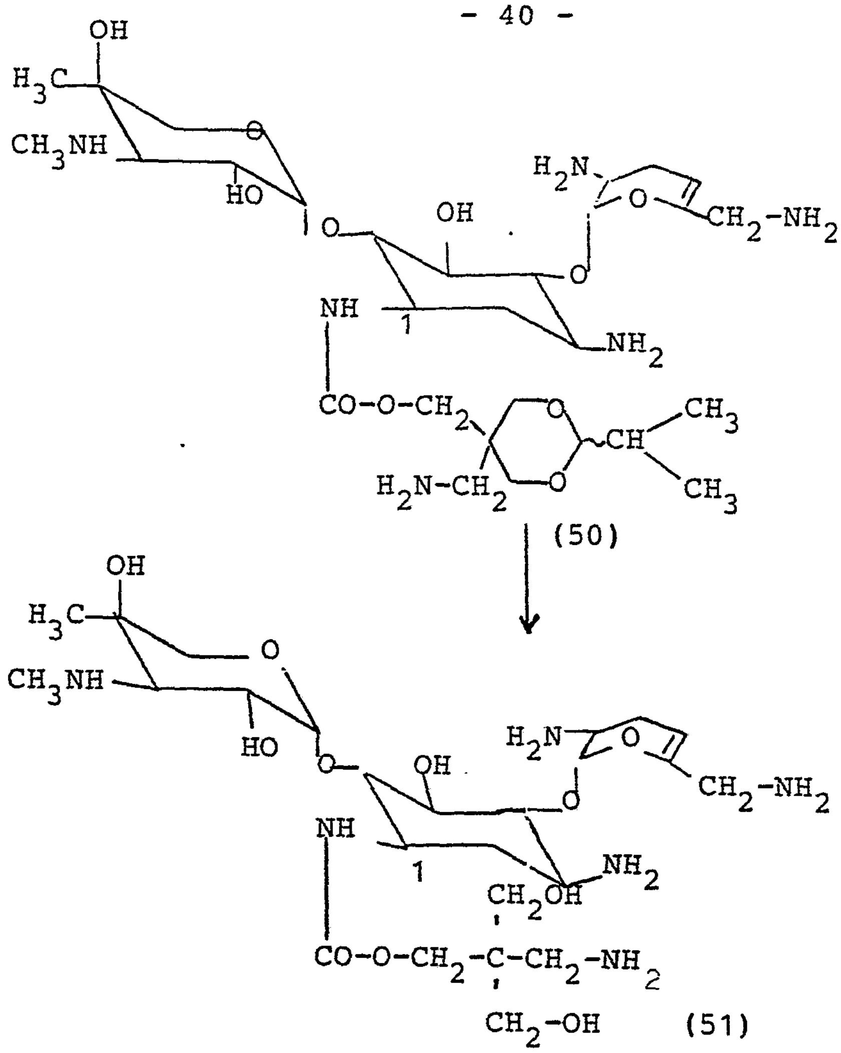

(50)

(51)

a) Herstellung der Verbindung (43)

Zu einer Suspension von 16,9 g $LiAlH_4$ (0,45 mol) in 150 ml Ether wird innerhalb 90 Minuten eine Lösung von 55,5 g (42) (J. Amer. Chem. Soc. 94, 171 [1972]) (0,20 mol) in 150 ml Ether getropft und 5 Stunden unter Rückfluß erhitzt. Man versetzt vorsichtig bei -5°C mit 20 ml Wasser, verdünnt mit 250 ml Ether, saugt ab und wäscht mit Ether nach. Der Niederschlag wird mit 300 ml Dioxan 3 Stunden ausgekocht und die vereinigten organischen Phasen mit $Na_2SO_4$ getrocknet, fil-

triert und eingeengt. Der feste Rückstand wird aus 20 ml Isopropanol umkristallisiert.

Ausbeute: 4,6 g (12,5%), Schmp. 99-100 °C.
NMR: Gemisch beider Konformeren.

b) Herstellung der Verbindungen (44), (45), (46)

4 g (0,02 mol) in 12 ml absol. Pyridin werden bei -15°C portionsweise mit 5 g p-Toluolsulfonsäurechlorid (0,025 mol) versetzt und bis zur homogenen Mischung gerührt. Man läßt bei 25 °C über Nacht stehen, engt im Vakuum ein und chromatographiert an 100 g Kieselgel mit System A.

Verbindung (46): 0,4 g öl; $R_f$-Wert (System A):0,84
Verbindung (44): 0,9 weißes Kristallisat; Schmp. 103-106°C; $R_f$-Wert (System A): 0,5
Verbindung (45): 1,0 g weißes Kristallisat; Schmp. 73-75°C; $R_f$-Wert (System A): 0,4

c) Herstellung der Verbindung (47)

0,9 g (2,6 x $10^{-3}$ mol) werden in 3 ml Dimethylformamid mit 390 mg $NaN_3$ in 0,5 ml Wasser versetzt und 5 Stunden zum Rückfluß erhitzt, eingeengt und über 50 g Kieselgel filtriert (Laufmittel: System A).

Ausbeute: 0,5 g öl (89%); IR: 2100/cm;
$R_f$-Wert (System A): 0,57.

d) Herstellung der Verbindung (48)

0,5 g (47) ($2,3 \times 10^{-3}$ mol) werden in 20 ml Essigester mit 0,3 g Pt-Mohr während 6 Tagen im $H_2$-Strom hydriert und die Abnahme der Ausgangsverbindung dünnschichtchromatographisch (System A) verfolgt. Nach Abfiltrieren des Katalysators wird im Vakuum eingeengt.

Ausbeute: 0,3 g (69 %).

e) Herstellung der Verbindung (49)

0,3 g (48) ($1,58 \times 10^{-3}$ mol) in 1,5 ml Dioxan und 0,75 ml 2n-NaOH werden bei pH $>$ 8 gleichzeitig mit 0,3 g o-Nitrophenylsulfenylchlorid ($1,5 \times 10^{-3}$ mol) in 1,5 ml Dioxan und 0,9 ml 2n-NaOH versetzt. Nach 15 Stunden wird im Vakuum eingeengt, in 5 ml $CH_2Cl_2$ aufgenommen, mit 2 mal 3 ml Wasser gewaschen, mit $Na_2SO_4$ getrocknet, eingeengt und über 50 g Kieselgel gereinigt (Laufmittel: System A).

Ausbeute: 0,4 g (74%);
$R_f$-Wert (System A): 0,42.

f) Herstellung der Verbindung (28)

Eine Lösung von 0,4 g (49) ($1,1 \times 10^{-3}$ mol) in 2 ml Pyridin wird mit 0,22 g Chlorameisensäure-p-nitrophenylester ($1,1 \times 10^{-3}$ mol) 5 Stunden

bei Raumtemperatur verrührt, im Hochvakuum eingeengt und an 50 g Kieselgel mit System A chromatographiert.

Ausbeute: 0,3 g (54%); IR: 1770/cm;
$R_f$-Wert (System A): 0,7.

g) Herstellung der Verbindungen (50) und (51)

Zu 165 mg 3,2',6',3"-Tetra-N-(o-nitrophenylsulfenyl)-sisomicin (30) ($1,5 \times 10^{-4}$ mol) in 0,75 ml Pyridin gibt man 91 mg (28) ($1,8 \times 10^{-4}$ mol), läßt über Nacht bei Raumtemperatur stehen und engt im Hochvakuum ein. Das Umsetzungsprodukt wird an 20 g Kieselgel mit System B abgetrennt, in 0,9 ml $CH_2Cl_2$ aufgenommen und zur Abspaltung der o-Nitrophenylsulfenyl-Schutzgruppen mit einer Lösung von 255 mg 2-Mercaptobenzthiazol ($1,5 \times 10^{-3}$ mol) in 0,9 ml Methanol/1,5 ml Methylenchlorid und bis zur sauren Reaktion mit etwas konzentrierter HCl versetzt und mit 1 ml Wasser verdünnt. Die saure Lösung blieb 2 Tage bei Raumtemperatur stehen, wobei aus dem primären Spaltprodukt die Isobutylidengruppe entfernt werden sollte. Da dünnschichtchromatographisch nach dieser Zeit das erste Spaltprodukt noch unverändert vorlag, wurde es zur Struktrurbestimmung durch Zugabe von Ionenaustauscher in der (OH-)-Form, Einengen und Chromatographie an 5 g Kieselgel (System C) isoliert.

Ausbeute: 43 mg farbloses Festprodukt;
$R_f$-Wert (System C): 0,62 (Sisomicin: $R_f$-Wert: 0,24).

Das $^{13}$C-NMR (s. Tabelle 3) zeigt das Vorliegen der Struktur (50) und damit der Isobutylidengruppe Ihre Abspaltung erfolgt durch erneutes Aufnehmen in 1,2 ml 6,5%iger Salzsäure und 2-tägiges Stehenlassen bei Raumtemperatur. Mit Ionenaustauscher in (OH-)-Form wird alkalisch gestellt, danach eingeengt und an 5 g Kieselgel chromatographiert (System C), wobei Verbindung (51) rein erhalten wird:

Ausbeute: 10 mg;
$R_f$-Wert (System C): 0,23
(Sisomicin: $R_f$-Wert: 0,24).

Tabelle 3:

$^{13}$C-Shifts δ (ppm, rel. zu TMS=O) für (50) in $D_2O$

| C-Atome, d.h. Zuordnung | Signallagen | rel. Intensitäten |
|---|---|---|
| C-1" | 100.665 | 3.916 |
| C-2" | 70.786 | 4.153 |
| C-3" | 70.609 | 2.401 |
| C-4" | 73.049 | 5.285 |
| C-5" | 68.571 | 3.577 |
| $N-CH_3$ an C-3" | 37.665 | 4.329 |
| $CH_3$ an C-4" | 22.533 | 3.108 |
| C-1 | 52.027 | 1.978 |
| C-2 | 32.546 | 5.679 |
| C-3 | 49.845 | 4.094 |
| C-4 | 81.818 | 1.801 |
| C-5 | 75.552 | 3.809 |
| C-6 | 84.442 | 2.779 |
| C-1' | 99.719 | 3.072 |
| C-2' | 47.341 | 4.233 |
| C-3' | 25.485 | 2.715 |
| C-4' | 97.665 | 4.341 |
| C-5' | 149.208 | 2.514 |
| C-6' | 43.105 | 4.841 |
| $(CH_3)_2$ vom Rest an N-1 | 16.788 | 8.721 |
| NH-COO vom Rest an N-1 | 158.675 | 2.167 |
| C-2 vom Rest an N-1 | 106.908 | 4.671 |
| C-4/C-6 vom Rest an N-1 | 64.255 | 4.215 |
| $OCH_2$ an C-5 vom Rest an N-1 | 69.631 | 3.422 |
| $CH_2NH_2$ an C-5 vom Rest an N-1 | 42.575 | 3.055 |
| C-5 vom Rest an N-1 | 38.210 | 5.400 |
| CH von Isopropyl vom Rest an N-1 | 35.611 | 2.061 |

Beispiel 5

1-N-(2-Amino-3-hydroxy-2-hydroxymethyl-propyloxycarbonyl)-sisomicin (52)

Reaktionsschema:

Reaktionsschema:

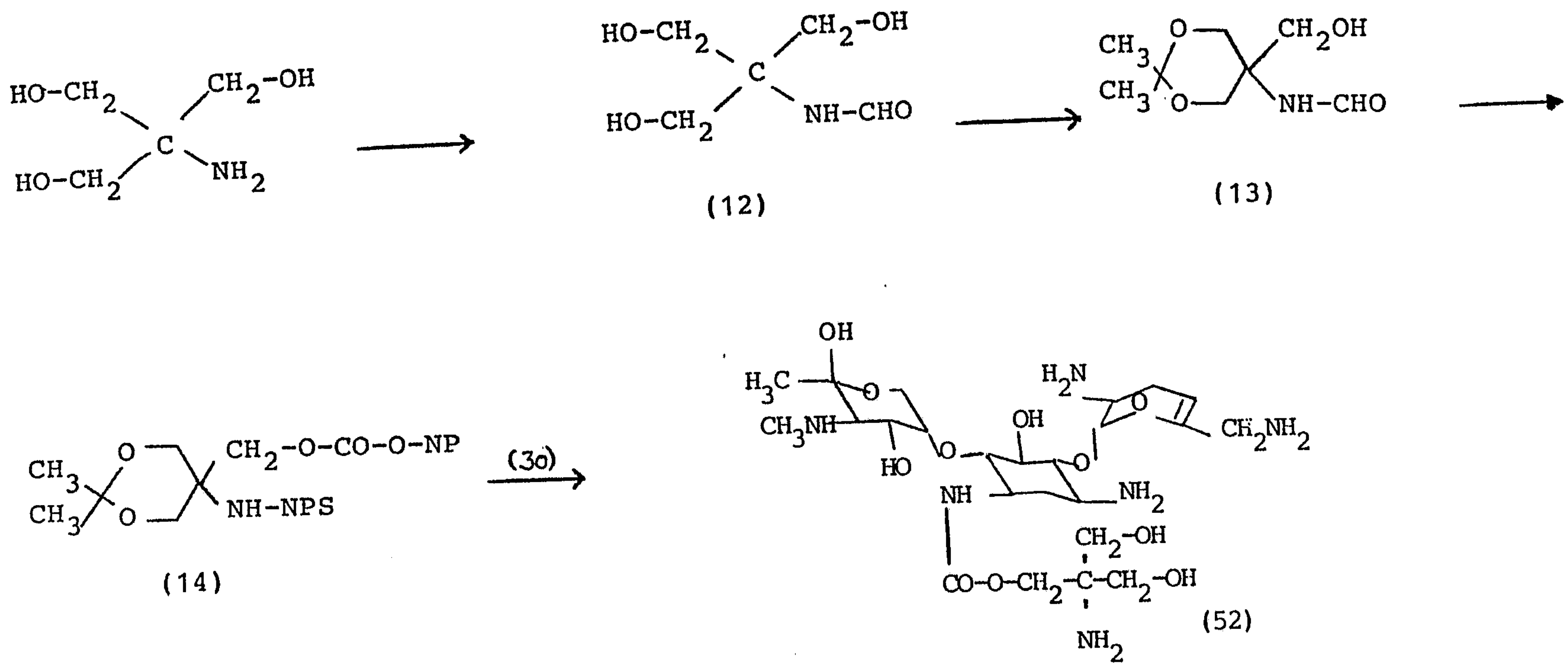


(12)

(13)

(14)

(52)

a) Herstellung der Verbindung (12)

60,5 g (0,5 mol) 2-Amino-2-hydroxymethyl-1,3-propandiol erhitzt man in 200 ml Methanol mit 42 g Ameisensäuremethylester (0,7 mol) 4 Stunden zum Rückfluß. Das beim Abkühlen ausfallende Kristallisat wird abgesaugt und mit Methanol gewaschen.

Ausbeute: 60,8 g (91,4%), Schmp. 117-118°C,
IR: 1670, 1645/cm.
NMR ($d_6$-DMSO): $\delta$ =~3,5 2 Signale ($3CH_2$), 4,83 s breit (3 OH), 6,83 d und 7,52 s breit (NH), 8,0 d und 8,2 d (CHO).

b) Herstellung der Verbindung (13)

29,8 g (12) (0,2 mol) werden in 150 ml Dimethylformamid mit 200 ml 2,2-Dimethoxypropan und 200 mg p-Toluolsulfonsäure 4 Stunden unter Rückfluß erhitzt, abgekühlt, in 1 Liter 0,5 % $NaHCO_3$-Lösung eingegossen und mit 3 mal 150 ml Methylenchlorid extrahiert. Die mit $Na_2SO_4$ getrocknete Lösung wird eingeengt und das zurückbleibende rotbraune Öl (3 g) mit System D an 100 g Kieselgel chromatographiert.

Ausbeute: 2,2 g (5,5%);
$R_f$-Wert (System D): 0,8;
IR: 1670/cm.

c) Herstellung der Verbindung (14)

3,8 g (13) (0,02 mol) erhitzt man mit einer Lösung von 10 g $Ba(OH)_2 \cdot 8\ H_2O$ in 50 ml Wasser 5 Stunden unter Rückfluß, engt ein und verrührt den Rückstand mit 5 ml System D. Die ungelösten Salze werden abgesaugt und das Filtrat an 100 g Kieselgel (System D) chromatographiert:

Ausbeute: 0,4 g (12,4%),
$R_f$-Wert (System D): 0,32.

300 mg (1,8 x $10^{-3}$mol) des so erhaltenen Amins werden in 2 ml Dioxan/1 ml 2n-NaOH durch gleichzeitiges Zutropfen von 355 mg NPS-Cl (1,8 x $10^{-3}$ mol) in 2 ml Dioxan und 1,3 ml 2n-NaOH bei Raumtemperatur acyliert. Das Produkt wird durch Einengen, Lösen in Methylenchlorid, Waschen mit Wasser, Trocknen mit $Na_2SO_4$ und Chromatographie (System A) an 40 g Kieselgel aufgearbeitet.

Ausbeute: 0,2 g gelbes Festprodukt (35%),
Schmp. 148-150°C;
$R_f$-Wert (System A): 0,15.

0,2 g (6,4 x $10^{-4}$ mol) des so erhaltenen acylierten Produkts werden in 1,5 ml Pyridin mit 188 mg Chlorameisensäure-p-nitrophenylester 4 Stunden bei Raumtemperatur umgesetzt. Man engt ein, nimmt in 5 ml Methylenchlorid auf, extrahiert mit 2 mal 1,5 ml Wasser und trocknet mit

$Na_2SO_4$. Das nach Chromatographie an 40 g Kieselgel (System A) isolierte Produkt (14) enthält neben der Hauptkomponente ($R_f$-Wert 0,48) noch Nitrophenol und eine weitere Verunreinigung ($R_f$-Wert 0,68) und kann direkt zur Weiterreaktion benutzt werden.

IR: 1760 $cm^{-1}$.

d) Herstellung der Verbindung (52)

165 mg 3,2', 6',3"-Tetra-N-(o-nitrophenylsulfenyl)-sisomicin (30) (1,5 x $10^{-4}$ mol) löst man in 0,75 ml Pyridin, versetzt mit 90 mg (14) und rührt 1 Tag bei Raumtemperatur. Die Aufarbeitung erfolgt analog Beispiel 1,f, wobei man das Endprodukt an 5 g Kieselgel (System C) chromatographisch reinigt.

Ausbeute: 21 mg (23,6%),
$R_f$-Wert (System C): 0,34 (Sisomicin $R_f$-Wert:0,25).

Beispiel 6

1-N-(1,4-Dihydroxy-3-n-propylamino-but-2-yloxycarbonyl)-sisomicin (53a)

Reaktionsschema:

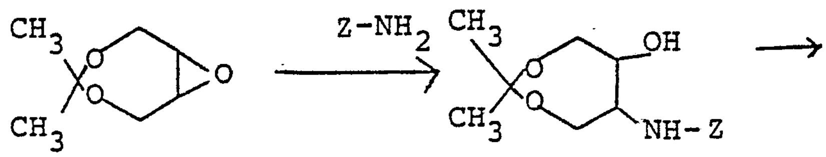


(8) (9)

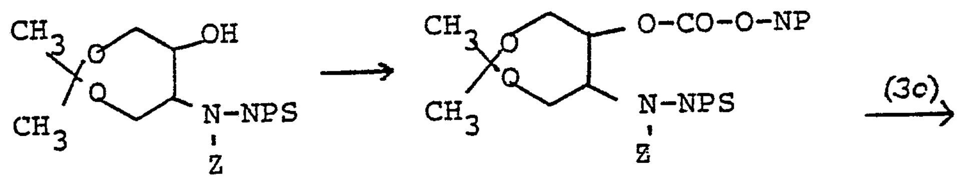


(10) (11)

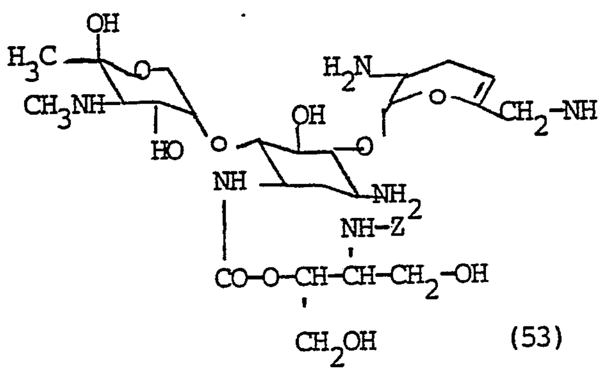

(53)

a: Z = $C_3H_7$

b: Z = H

c: Z = $n\text{-}C_4H_9$

d: Z = $CH_2CH_2\text{-}O\text{-}C(C_6H_5)_3$

e: Z = $CH_2CH_2\text{-}OH$

a) Herstellung der Verbindung (9a) (mit $Z=n-C_3H_7$)

5.76 g (0,04 mol) 4,4-Dimethyl-3,5,8-trioxabicyclo /5.1.0/octan (8) (J.Org. Chem. 41, 2469 /1976/) werden in 20 ml Methylenchlorid mit 2,4 g n-Propylamin 10 Stunden unter Rückfluß erhitzt und anschließend bei 40°C im Vakuum eingeengt.

Ausbeute: 7,3 g Öl (90%); Rf-Wert (System D): 0,33.

b) Herstellung der Verbindung (10a)

Eine Lösung von 1 g (9a) (0,005 mol) in 7 ml Dioxan versetzt man gleichzeitig bei pH >8 mit einer Lösung von 0,95 g NPS-Cl in 2.5 ml Dioxan und 2.5 ml 2n-NaOH. Man rührt einige Stunden bei Raumtemperatur, engt im Vakuum ein, nimmt in Methylenchlorid auf, wäscht mit Wasser, trocknet mit $Na_2SO_4$ und dampft ein. Zur Reinigung chromatographiert man über 60 g Kieselgel mit Toluol als Laufmittel.

Ausbeute: 1 g oranges Öl, Rf-Wert (System A): 0,4

c) Herstellung der Verbindung (11a)

0,89 (10a) (0,0025 mol) werden in 7,5 ml Pyridin mit 0,5 g Chlorameisensäure-p-nitrophenylester umgesetzt und analog Beispiel 1e aufgearbeitet.

Ausbeute: 1,1 g (84%); Rf-Wert (System A): 0,76;
IR: 1770/cm.

d) Herstellung der Verbindung (53a)

Eine Lösung von 165 mg ($1,5 \times 10^{-4}$ mol) 3,2',6',3"-Tetra-N-(o-nitrophenylsulfenyl)-sisomicin (30) in 0,75 ml Pyridin werden mit 90 mg (11a) umgesetzt und entsprechend Beispiel 1f aufgearbeitet. Die Abspaltung der Isopropyliden-Schutzgruppe war nach 2-stündigem Stehen der sauren wäßrigen Lösung quantitativ.

Ausbeute: 20 mg (21%); Rf-Wert (System C): 0,51
(Sisomicin: 0,24).

Beispiel 7

1-N-(1,4-Dihydroxy-3-amino-but-2-yloxycarbonyl)-sisomicin (53b)

Die Herstellung erfolgt entsprechend Beispiel 6 unter Verwendung von $NH_3$ über die Stufen (9b) (Rf-Wert [System D] = 0,20), (10b) (Rf-Wert [System A] : 0,20) und (11b) (Rf-Wert [System A] = 0,68; IR= 1780/cm) in einer Ausbeute von 18%. Rf-Wert (System C): 0,27 (Sisomicin: 0,25).

Beispiel 8

1-N-(1,4-Dihydroxy-3-n-butylamino-but-2-yloxycarbonyl)-sisomicin (53 c)

Die Herstellung erfolgt entsprechend Beispiel 6 unter Verwendung von n-Butylamin über die Stufen (9c) (Rf-Wert /System D/: 0,57; Schmelpunkt 156°), (10c) (Rf-Wert /System A/: 0,37) und (11c) (IR: 1780/cm) in einer Ausbeute von 30 %; Rf-Wert (System C): 0,54.

Beispiel 9

1-N-/1.4-Dihydroxy-3-(2-hydroxyethylamino)-but-2-yloxycarbonyl/-sisomicin (53 e)

Die Herstellung erfolgt entsprechend Beispiel 6 unter Verwendung von O-Tritylethanolamin über die Stufen (9d) (Rf-Wert /System D/: 0,7), (10d) (Rf-Wert /System A/: 0,42) und (11 d) (Rf-Wert /System A/: 0,92) zur Verbindung (53d), wobei die O-Tritylschutzgruppe im letzten Schritt durch 15-stündiges Stehenlassen der sauren wäßrigen Lösung unter Bildung der Verbindung (53e) abgespalten wird.

Ausbeute: 49%, Rf-Wert (System C): 0,38.

Patentansprüche

1) Verbindungen der allgemeinen Formel

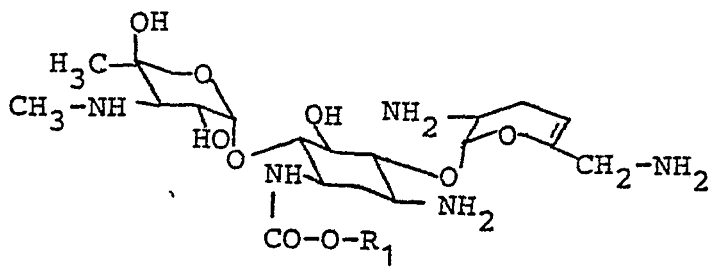

(I)

in welcher

$R_1$ für einen Rest der Formel -A-XH steht, wobei

A für einen gegebenenfalls verzweigten Alkylen- oder Alkenylenrest mit 3 bis 7 C-Atomen steht, der durch 2 bis 4 Hydroxylgruppen substituiert ist,

X Sauerstoff oder $-NR_2-$ bedeutet und

$R_2$ Wasserstoff, eine Alkyl- oder Hydroxyalkylgruppe mit 1 bis 6 C-Atomen oder eine Aralkylgruppe mit 7 bis 10 C-Atomen darstellt,

und deren pharmazeutisch verwendbare Salze.

2) Verbindungen nach Anspruch 1, dadurch gekennzeichnet, daß

A für einen gegebenenfalls verzweigten Alkylrest mit 4 bis 6 C-Atomen steht, der durch 2 bis 4 Hydroxylgruppen substituiert ist.

3) Verbindungen nach Anspruch 1, dadurch gekennzeichnet, daß

X für eine Gruppe $-NR_2-$ steht, wobei

$R_2$ Wasserstoff, eine Alkyl- oder Hydroxyalkylgruppe mit 2 bis 4 C-Atomen oder einen Benzylrest darstellt.

4) Verbindungen nach Anspruch 1, dadurch gekennzeichnet, daß $R_1$ für einen der Reste

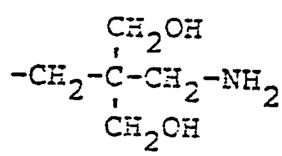

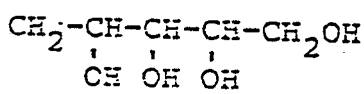

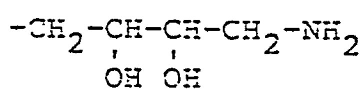

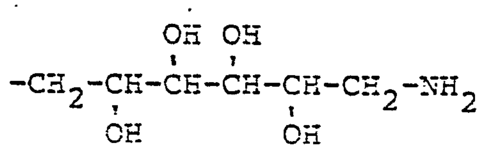

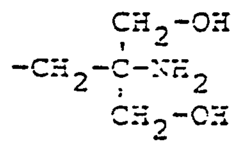

oder

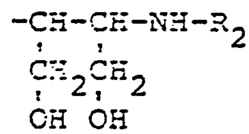

steht, wobei

$R_2$ H, $C_3H_7$, $C_4H_9$, $CH_2-C_6H_5$ oder $CH_2-CH_2-OH$

darstellt.

5) Verfahren zur Herstellung von Verbindungen nach Anspruch 1, dadurch gekennzeichnet, daß man eine Verbindung der Formel II

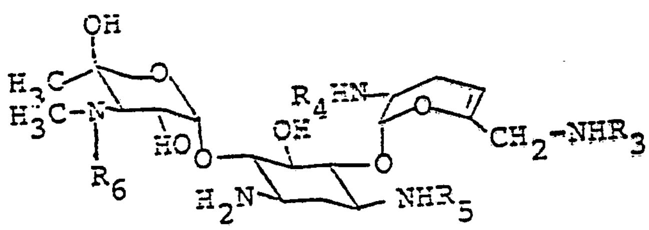

(II)

worin
$R_3$, $R_4$ und $R_5$ für $-CO-R_7$ oder $-S-R_8$ und
$R_6$ für $-S-R_8$ stehen,
wobei $R_7$ einen Rest der Formeln

$-CHal_3$, $-(CH_2)_{n_1}-B$, $-O-E$,

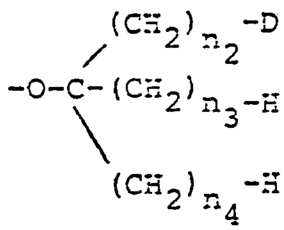

oder

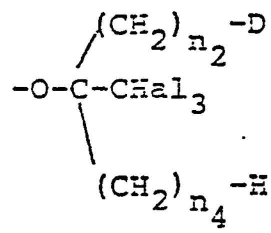

B und D Wasserstoff oder gegebenenfalls substituiertes Phenyl

E gegebenenfalls substituiertes Phenyl,
$n_1$ eine Zahl von 0 - 5,
$n_2$, $n_3$ und $n_4$ eine Zahl von 0 - 5,
Hal Fluor, Chlor oder Brom und
$R_8$ gegebenenfalls substituiertes Phenyl, Di- oder Triphenylmethyl

bedeuten,

in an sich bekannter Weise mit einem Acylierungsmittel der Formel III

G-CO-O-R' (III)

worin

G eine Abgangsgruppe darstellt und R' für einen Rest $R_1$ steht, in dem gegebenenfalls vorhandene Aminogruppen und die

Hydroxylgruppen durch geeignete Schutzgruppen geschützt sind, umsetzt, die Schutzgruppen $R_3$, $R_4$, $R_5$, $R_6$, sowie gegebenenfalls Tetrahydropyranylreste oder Alkylidengruppen, in üblicher Weise abspaltet und die resultierenden Verbindungen gegebenenfalls in ihre pharmazeutisch verwendbaren Salze überführt, wobei $R_1$ die in den Ansprüchen 1 bis 4 angegebene Bedeutung hat.

6) Verfahren nach Anspruch 5, dadurch gekennzeichnet, daß man als Verbindung der Formel (II) 2',3,3",6'-Tetra-N-(o-nitrophenylsulfenyl)-sisomicin, 3"-N-(o-Nitrophenylsulfenyl)-2'-3,6'-tris-N-trichloracetyl-sisomicin, 3"-N-(o-Nitrophenylsulfenyl)-2',3,6'-tris-N-trifluoracetyl-sisomicin, 3"-N-(o-Nitrophenylsulfenyl)-2',3,6'-tris-N-(2,2,2-trichlorethoxycarbonyl)-sisomicin, 3"-N-(o-Nitrophenylsulfenyl)-2',3,6'-tris-N-(1,1-dimethyl-2,2,2-trichlorethoxycarbonyl)-sisomicin, 3"-N-(o-Nitrophenylsulfenyl)-2',3,6'-tris-N-(4-methoxybenzyloxycarbonyl)-sisomicin, 3"-N-(o-Nitrophenylsulfenyl)-2',3,6'-tris-N-phenoxycarbonylsisomicin oder 3"-N-(o-Nitrophenylsulfenyl)-2',3,6'-tris-N-(tert.-butoxycarbonyl)-sisomicin einsetzt.

7) Arzneimittel, enthaltend als Wirkstoff eine Verbindung gemäß Anspruch 1 bis 4.

8) Verwendung von Verbindung gemäß Anspruch 1 bis 4 bei der Bekämpfung von durch Bakterien hervorgerufenen Krankheiten.

0056575

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung
EP 82 10 0038

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| Y | EP - A - 0 019 843 (BAYER)<br>* Seiten 44-48 *<br>--- | 1,7 |
| Y | EP - A - 0 007 996 (BAYER)<br>* Seiten 38-46 *<br>--- | 1,7 |
| Y | EP - A - 0 002 450 (BAYER)<br>* Seiten 77-90 * | 1,7 |
| D | & DE - A - 2 753 769<br>--- | |
| Y | FR - A - 2 264 553 (SCHERICO)<br>* Seiten 34-39 *<br>& DE - A - 2 502 296<br>--- | 1,7 |
| Y | DE - A - 2 708 008 (PFIZER CORP)<br>* Seiten 1-4 *<br>--- | 1,7 |
| Y | CHEMICAL ABSTRACTS, Band 87, 1977, Seite 503, Zusammenfassung 53534e, COLUMBUS, OHIO (US)<br>./.. | |

KLASSIFIKATION DER ANMELDUNG (Int Cl. )

C 07 H 15/22
A 61 K 31/70//
C 07 D 317/24
C 07 D 317/28
C 07 D 317/32
C 07 D 317/20
C 07 D 329/06
C 07 D 321/06

RECHERCHIERTE SACHGEBIETE (Int Cl.³)

C 07 H 15/22
A 61 K 31/70

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-7

Unvollständig recherchierte Patentansprüche:

Nicht recherchierte Patentansprüche: 8

Grund für die Beschränkung der Recherche:

Verfahren zur chirurgischen oder therapeutischen Behandlung des menschlichen oder tierischen Körpers (siehe Art. 52(4) des Europäischen Patentübereinkommens).

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| Den Haag | 28.01.1982 | VERHULST |

Europäisches Patentamt

**EUROPÄISCHER TEILRECHERCHENBERICHT**

0056575

Nummer der Anmeldung

EP 82 10 0038

- 2 -

| EINSCHLÄGIGE DOKUMENTE | | | KLASSIFIKATION DER ANMELDUNG (Int Cl.) |
|---|---|---|---|
| Kategorie | Kennzeichnung des Dokuments mit Angabe, soweit erforderlich der maßgeblichen Teile | betrifft Anspruch | |
| | & JP - A - 76 143 644 (TEIJIN LTD.), 10. Dezember 1976<br><br>* insgesamt *<br><br>----------- | 1,7 | |
| | | | RECHERCHIERTE SACHGEBIETE (Int. Cl.3) |